# EUROPEAN PATENT APPLICATION

(11) **EP 4 846 929 A1**
(43) Date of publication of application: **02.12.2026**
(21) Application number: 26181723.3
(22) Date of filing: 28.05.2026
(51) Int. Cl.: A61B 5/00, A61B 5/291, A61B 5/372, G16H 30/40

(54) **SYSTEMS AND METHODS OF DETERMINING ANATOMICAL SOURCES OF ABNORMAL NEUROLOGICAL EVENTS**

(30) Priority: 28.05.2025 US 202563813502 P
(71) Applicant: Cadwell Laboratories, Inc., Richland, WA 99336 (US)
(72) Inventor: Cadwell, John A., Richland, 99352 (US)
(74) Representative: Murgitroyd & Company

(57) **Abstract**

A method of assisting a clinician in determining a neural source of an abnormal or seizure event includes visualization of EEG data in association with a visual representation of corresponding electrodes and contacts in a patient's 3D brain model. A module is configured to generate and use 3D ROI (region of interest) montaging and determine data indicative of a plurality of largest measured/recorded EEG voltages, corresponding to EEG traces associated with an abnormal or seizure event, in order to model a best-fitting vector dipole source responsible for the abnormal or seizure event. Subsequently, the vector dipole source is employed to a) select electrode contacts in the vicinity of the vector dipole source for montage generation and/or b) model virtual electrodes that may be more evenly distributed around the vector dipole source to facilitate montage generation.

## Description

### CROSS-REFERENCE

The present application relies on, for priority, United States Patent Provisional Application Number 63/813,502, titled "Systems and Methods of Determining a Neural Source of an Abnormal Event by Analyzing Electrophysiological Data Integrated with a Visual Representation of Associated Electrodes and Contacts in a Patient-Specific Three-Dimensional Brain Model" and filed on May 28, 2025, which is herein incorporated by reference in its entirety

### FIELD

The present specification is related generally to the field of diagnostic systems. More specifically, the present specification is related to integrating electrophysiological data with a visual representation of correlated electrodes and contacts in a patient-specific three-dimensional brain model to determine a neural source of an abnormal or seizure event.

### BACKGROUND

Electroencephalography (EEG) records the brain's activity as a graphical display of time-varying voltages. These voltages can be represented as either an absolute value at each electrode contact relative to a reference point (referential) or as the voltage difference between pairs of electrodes (differential or bipolar montage). The electrical activity recorded in an EEG originates from groups of cortical neurons, with their combined activity measurable at different electrode sites. When a small cluster of neurons, such as those in an epileptic focus, discharges synchronously, a transient spike may appear on the recording, overlaying the continuous background EEG activity. This spike can be modeled as a single dipole, representing a signal with a specific directional orientation.

The strength of the electrical signal detected by EEG electrodes depends on their alignment and proximity to the dipole source. Electrodes that are aligned with the dipole's orientation register the strongest signals, while those positioned perpendicular to the dipole present the weakest signals. Electrodes located closer to the dipole record signals of greater amplitude, and the polarity observed at each electrode varies according to its relative orientation with respect to the dipole's direction.

The selection of contacts or differential electrode pairs, impacts EEG signal detection, as the spatial configuration determines sensitivity to underlying dipole sources. By analyzing multiple sets of differential pairs, clinicians can estimate dipole characteristics, such as position and orientation and neural source (the associated group of cortical cells responsible for generating the dipole) location. Clinicians can perform a visual analysis, which involves identifying dipole patterns by assessing signal polarity and amplitude variations across montages or by addressing the 'inverse problem' mathematically, which involves determining the presumed location, orientation, and voltage of a dipole that would produce the actual signals recorded by the contacts.

EEG analysis requires the strategic selection of differential electrode pairs/contacts to visually or mathematically determine the location or origin of an abnormal event or spike event (such as an epileptic spike). Ideally, the set includes primary electrode pairs, which are closest to the neural source and exhibit the strongest amplitude due to their proximity to the abnormal event. Further proximity to the source enhances signal clarity and reduces ambiguity in spike location. The set may also include secondary electrode pairs, that are approximately orthogonal to the primary set and are used to establish a coordinate system.

Extracranial (scalp) EEG systems employ standardized electrode placement protocols to optimize signal acquisition or analysis. A plurality of electrodes, typically 20-21, are positioned using conventional systems such as the 10-20 system (spacing electrodes 10% or 20% of head dimensions), 10-10, or 10-05, which enhance reproducibility and coverage. With 21 electrodes, the total number of potential discrete differential pairs is calculated as [n(n-1)]/2 = 210 (20+19+18+...+1). Using 21 common-reference pairs (referencing each electrode to a common point) in this equation yields approximately 231 configurations. Clinicians may prioritize adjacent electrode pairs to create montages that emphasize local signal gradients and minimize noise. Multiple montages with varied orientations are applied iteratively to triangulate abnormal activity.

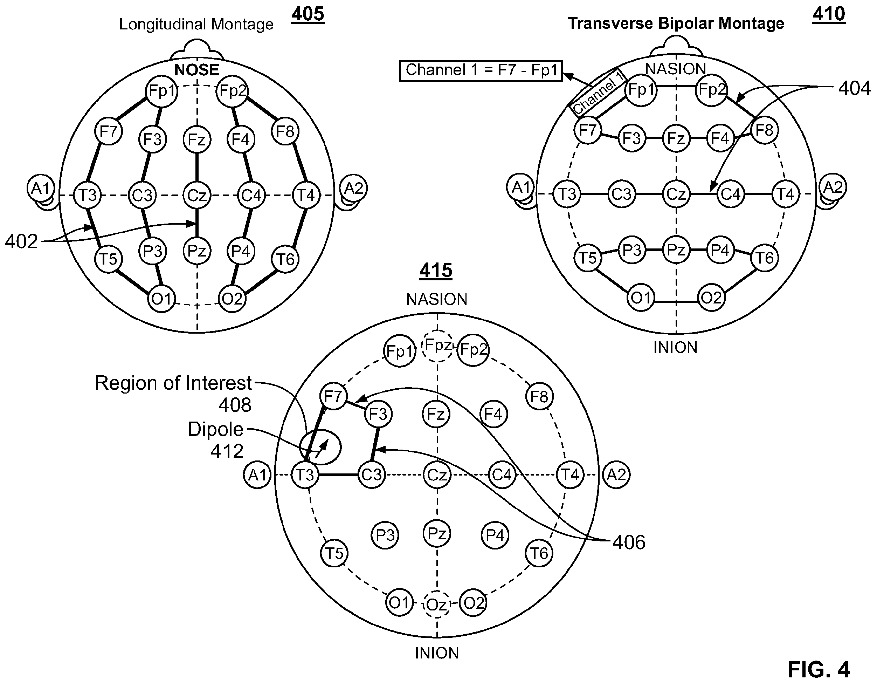

FIG. 4 illustrates exemplary montages strategies for localizing neural dipoles, based on the standard 10-20 system of placing electrode contacts on the scalp of a patient. View 405 shows a first set of montages 402 corresponding to longitudinal bipolar montaging (electrode pairs are aligned along the anterior-posterior axis) and view 410 shows a second set of montages 404 corresponding to transverse bipolar montaging (pairs are arranged laterally to capture left-right dipole orientations). When an abnormal EEG event, such as a spike, occurs, it is detected by electrode contacts near the neural source and are represented by montages that include those contacts. The spike originates from the electrical field generated by a presumed dipole, representing neuronal activity. An optimal montage 406, as shown in view 415, would focus on a region of interest 408 encompassing the presumed dipole 412, combining longitudinal and transverse electrode pairs to capture spatial orientation and amplitude gradients. However, instrumentation limitations restrict the number of channels that can be displayed simultaneously, requiring clinicians to manually alternate between montages during analysis.

Stereo EEG is an invasive monitoring technique in which multiple depth electrodes, each containing several contacts, are surgically inserted through burr holes into the brain, targeting regions of interest previously identified by noninvasive methods such as extracranial EEG analyses. The main goal of stereo EEG is to improve the precision of localizing abnormal brain areas for therapeutic interventions. Typically, this procedure involves between 200 and 300 contacts, which theoretically allows for 20,000 to 45,000 possible montage configurations. To manage this complexity, clinicians often use either differential pairs of sequential contacts along each electrode or referential pairs that compare each contact to a common reference point. This strategy reduces the number of montage channels to two sets, each comprising 200 to 300 channels. However, even with these approaches, neither montage configuration is guaranteed to optimally represent every specific abnormal event detected during monitoring.

In extracranial (scalp) EEG, clinicians can estimate regions of interest using 20-channel recordings by visually analyzing EEG traces with standard montages, as electrodes are uniformly distributed on a 2D scalp surface. In contrast, stereo EEG involves depth electrodes placed in 3D brain space, where contact locations lack intuitive spatial correlations across electrodes. With approximately 400 channels organized by sequential contacts along each electrode, abnormal signals often span multiple electrodes, complicating the mapping of these signals to their originating brain volumes. The dispersed 3D arrangement and non-uniformity make it difficult to visually infer the neural source, as spike patterns may reflect complex dipole interactions across disparate regions.

There is a need for systems and methods that can generate topographic views of stereotactic electroencephalography (EEG) activity that enhance visualization and localization of abnormal or seizure activity within a specific region of interest (ROI). There is also a need for algorithmic approaches that can create virtual contacts in order to assist in determining the neural source of abnormal or seizure activity.

### SUMMARY

The following embodiments and aspects thereof are described and illustrated in conjunction with systems, tools and methods, which are meant to be exemplary and illustrative, and not limiting in scope. The present application discloses numerous embodiments.

In some embodiments, the present specification describes a computer-implemented method of enabling a user to generate one or more montages using a computing device, wherein the computing device comprises at least one processor configured to execute programmatic instructions and a non-transient memory adapted to store the programmatic instructions, comprising: generating a graphical user interface, wherein the graphical user interface comprises at least a first display area and a second display area, wherein the first and second display areas are separate and positioned side-by-side; acquiring, using the computing device, post-surgical data, wherein the post-surgical data comprises a three-dimensional image of a patient's brain and comprises data indicative of a visual representation of one or more electrodes placed in the patient's brain, wherein each of the one or more electrodes has associated one or more contacts therewith; acquiring, using the computing device, EEG data of the patient, wherein the EEG data comprises a first plurality of EEG traces; simultaneously displaying the EEG data in the first display area and the post-surgical data in the second display area of the graphical user interface; automatically visually associating the visual representation of each of the one or more electrodes and associated one or more contacts in the post-surgical data with a corresponding one of the first plurality of EEG traces; receiving a user selection of at least one EEG trace of the first plurality of EEG traces; in response to said receiving of the user selection of the at least one EEG trace, causing at least one contact of the one or more contacts to be automatically selected in the second display area, wherein said automatically selected at least one contact is associated with said user-selected at least one EEG trace; receiving a user selection of a region of interest around the automatically selected at least one contact; in response to said receiving of the user selection of the region of interest, causing a first plurality of contacts, within the region of interest, to be automatically selected; automatically generating a first set of montages based on the automatically selected first plurality of contacts; and displaying the automatically generated first set of montages in the first display area.

Optionally, the three-dimensional image is co-registered with a three-dimensional coordinate system

Optionally, the method further comprises automatically computing a lead field matrix using a head model.

Optionally, the method further comprises generating the head model by associating a volume conductivity of the patient's brain with the three-dimensional image of the patient's brain.

Optionally, the lead field matrix defines an electric potential that a unit dipole would produce at each of the first plurality of contacts.

Optionally, the method further comprises automatically generating an initial vector dipole source using data indicative of a first plurality of voltages associated with said at least one EEG trace of the first plurality of EEG traces.

Optionally, the method further comprises automatically determining a plurality of expected voltages generated by the initial vector dipole source at at least a portion of the first plurality of contacts within the region of interest.

Optionally, the method further comprises automatically computing a computed dipole source based on said plurality of expected voltages.

Optionally, the method further comprises determining the computed vector dipole source by: generating a composite error value by comparing the plurality of expected voltages with a plurality of actual voltages at the at least a portion of the first plurality of contacts within the region of interest; iteratively changing dimensions of the initial vector dipole source to generate successive vector dipole sources; and iteratively generating a successive composite error value for each of the successive vector dipole sources in a direction of decreasing composite error value until a minimum composite error is determined, wherein the computed vector dipole source corresponds to the minimum composite error.

Optionally, the computed vector dipole source is displayed in the second display area.

Optionally, each of the initial vector dipole source and the computed vector dipole source is associated with a position, an orientation and an amplitude.

Optionally, the method further comprises automatically generating a plurality of virtual contacts based on the computed dipole source, wherein the plurality of virtual contacts are displayed in the second display area.

Optionally, the method further comprises automatically generating a second set of montages based on the plurality of virtual contacts, wherein the second set of montages is displayed in the second display area, and wherein a second plurality of EEG traces, corresponding to the second set of montages, is displayed in the first display area.

Optionally, the selected at least one EEG trace corresponds to an abnormal event.

Optionally, the method further comprises enabling the region of interest to be specified by presenting a tool adapted to draw a 3D geometrical shape around the automatically selected at least one contact, and wherein at least a size of the 3D geometrical shape determines automatic selection of the first plurality of contacts.

Optionally, the first plurality of contacts is positioned within an electrode and/or across electrodes.

Optionally, the method further comprises automatically generating a third set of montages based on a third plurality of contacts positioned in the vicinity of the computed dipole source; and displaying a third plurality of EEG traces corresponding to the third set of montages in the first display area.

In some embodiments, the present specification is directed towards a computer-implemented method of enabling a user to generate one or more montages using a computing device, wherein the computing device comprises at least one processor configured to execute programmatic instructions and a non-transient memory adapted to store the programmatic instructions, comprising: generating a graphical user interface, wherein the graphical user interface comprises at least a first display area and a second display area, wherein the first display area is separate and distinct from the second display area, and wherein the first and second display areas are positioned side-by-side; acquiring, using the computing device, post-surgical data, wherein the post-surgical data comprises a three-dimensional image of a patient's brain and comprises data indicative of a visual representation of one or more electrodes surgically placed in the patient's brain, wherein each of the one or more electrodes has associated one or more contacts, and wherein the three-dimensional image is co-registered with a three-dimensional coordinate system; acquiring, using the computing device, EEG data of the patient; displaying, simultaneously, the EEG data in the first display area and the post-surgical data in the second display area of the graphical user interface, wherein the EEG data is displayed as a first plurality of EEG traces; automatically visually associating the visual representation of each of the one or more electrodes and associated one or more contacts in the post-surgical data with a corresponding one of the first plurality of EEG traces; automatically selecting at least one contact in the second display area in response to receiving user data indicative of at least one EEG trace of the first plurality of EEG traces, wherein said automatically selected at least contact is associated with said selected at least one EEG trace; automatically selecting a first plurality of contacts, within the region of interest, in response to receiving user data indicative of a region of interest around the automatically selected at least one contact; automatically generating a first set of montages based on the first plurality of contacts; automatically computing a lead field matrix using the head model; automatically generating an initial vector dipole source using data indicative of a first plurality of voltages associated with said at least one EEG trace of the first plurality of EEG traces; automatically determining a plurality of expected voltages generated by the initial vector dipole source at at least a portion of the first plurality of contacts within the region of interest; automatically computing a computed dipole source based on said plurality of expected voltages; automatically generating a plurality of virtual contacts based on the computed vector dipole source, wherein the plurality of virtual contacts are displayed in the second display area; and automatically generating a second set of montages based on the plurality of virtual contacts and displaying a second plurality of EEG traces, corresponding to the second set of montages, in the first display area.

Optionally, the method further comprises automatically generating a third set of montages based on a third plurality of contacts positioned in a vicinity of the computed vector dipole source.

Optionally, the lead field matrix defines an electric potential that a unit dipole would produce at each of the first plurality of contacts.

Optionally, each of the initial vector dipole source and the computed vector dipole source is associated with a position, an orientation and an amplitude.

Optionally, the method further comprises generating the head model by associating a volume conductivity of the patient's brain with the three-dimensional image of the patient's brain.

In some other embodiments, the present specification discloses a computer-implemented method of enabling a user to generate one or more montages using a computing device, wherein the computing device comprises at least one processor configured to execute programmatic instructions and a non-transient memory adapted to store the programmatic instructions, comprising: generating a graphical user interface, wherein the graphical user interface comprises at least a first display area and a second display area, wherein the first display area is separate and distinct from the second display area, and wherein the first and second display areas are positioned side-by-side; acquiring, using the computing device, post-surgical data, wherein the post-surgical data comprises a three-dimensional image of a patient's brain and comprises data indicative of a visual representation of one or more electrodes surgically placed in the patient's brain, wherein each of the one or more electrodes has associated one or more contacts, and wherein the three-dimensional image is co-registered with a three-dimensional coordinate system; acquiring, using the computing device, EEG data of the patient; displaying, simultaneously, the EEG data in the first display area and the post-surgical data in the second display area of the graphical user interface, wherein the EEG data is displayed as a first plurality of EEG traces; automatically visually associating the visual representation of each of the one or more electrodes and associated one or more contacts in the post-surgical data with a corresponding one of the first plurality of EEG traces; selecting, by the user, at least one EEG trace of the first plurality of EEG traces thereby causing at least one contact to be automatically selected in the second display area, wherein said automatically selected at least one contact is associated with said selected at least one EEG trace; specifying, by the user, a region of interest around the automatically selected at least one contact thereby causing a first plurality of contacts, within the region of interest, to be automatically selected; automatically generating a first set of montages based on the first plurality of contacts; automatically computing a lead field matrix using a head model; automatically generating an initial vector dipole source using data indicative of a first plurality of voltages associated with said at least one EEG trace of the first plurality of EEG traces; automatically determining a plurality of expected voltages generated by the initial vector dipole source at at least a portion of the first plurality of contacts within the region of interest; automatically computing a computed dipole source based on said plurality of expected voltages; automatically generating a plurality of virtual contacts based on the computed dipole source, wherein the plurality of virtual contacts are displayed in the second display area; and automatically generating a second set of montages based on the plurality of virtual contacts, wherein the second set of montages is displayed in the second display area, and wherein a second plurality of EEG traces, corresponding to the second set of montages, is displayed in the first display area.

Optionally, the selected at least one EEG trace corresponds to an abnormal event.

Optionally, the region of interest is specified by drawing a 3D geometrical shape around the automatically selected at least one contact, wherein at least a size of the 3D geometrical shape determines automatic selection of the first plurality of contacts.

Optionally, the first plurality of contacts is positioned within an electrode and/or across electrodes.

Optionally, the lead field matrix describes an electric potential that a unit dipole would produce at each of the first plurality of contacts.

Optionally, each of the initial vector dipole source and the computed vector dipole source is associated with a position, an orientation and an amplitude.

Optionally, the head model is generated by associating a volume conductivity of the patient's brain with the three-dimensional image of the patient's brain.

Optionally, the computed vector dipole source is determined by: generating a composite error value by comparing the plurality of expected voltages with a plurality of actual voltages at at least a portion of the first plurality of contacts within the region of interest; iteratively changing dimensions of the initial vector dipole source to generate successive vector dipole sources; and iteratively generating a successive composite error value for each of the successive vector dipole sources in a direction of decreasing composite error value until a minimum composite error is determined, wherein the computed vector dipole source corresponds to the minimum composite error.

Optionally, the computed vector dipole source is displayed in the second display area.

Optionally, the computer-implemented method further comprises: automatically generating a third set of montages based on a third plurality of contacts positioned in the vicinity of the computed vector dipole source; displaying the third set of montages in the second display area; and displaying a third plurality of EEG traces in the first display area, wherein the third plurality of EEG traces correspond to the third set of montages.

In still some other embodiments, the present specification also discloses a computer-implemented method of enabling a user to generate one or more montages using a computing device, wherein the computing device comprises at least one processor configured to execute programmatic instructions and a non-transient memory adapted to store the programmatic instructions, comprising: generating a graphical user interface, wherein the graphical user interface comprises at least a first display area and a second display area, wherein the first display area is separate and distinct from the second display area, and wherein the first and second display areas are positioned side-by-side; acquiring, using the computing device, post-surgical data, wherein the post-surgical data comprises a three-dimensional image of a patient's brain and comprises data indicative of a visual representation of one or more electrodes surgically placed in the patient's brain, wherein each of the one or more electrodes has associated one or more contacts, and wherein the three-dimensional image is co-registered with a three-dimensional coordinate system; acquiring, using the computing device, EEG data of the patient; displaying, simultaneously, the EEG data in the first display area and the post-surgical data in the second display area of the graphical user interface, wherein the EEG data is displayed as a first plurality of EEG traces; automatically visually associating the visual representation of each of the one or more electrodes and associated one or more contacts in the post-surgical data with a corresponding one of the first plurality of EEG traces; selecting, by the user, at least one EEG trace of the first plurality of EEG traces thereby causing at least one contact to be automatically selected in the second display area, wherein said automatically selected at least contact is associated with said selected at least one EEG trace; specifying, by the user, a region of interest around the automatically selected at least one contact thereby causing a first plurality of contacts, within the region of interest, to be automatically selected; automatically generating a first set of montages based on the first plurality of contacts; automatically computing a lead field matrix using the head model; automatically generating an initial vector dipole source using data indicative of a first plurality of voltages associated with said at least one EEG trace of the first plurality of EEG traces; automatically determining a plurality of expected voltages generated by the initial vector dipole source at at least a portion of the first plurality of contacts within the region of interest; automatically computing a computed dipole source based on said plurality of expected voltages; automatically generating a plurality of virtual contacts based on the computed vector dipole source, wherein the plurality of virtual contacts are displayed in the second display area; automatically generating a second set of montages based on the plurality of virtual contacts, wherein the second set of montages is displayed in the second display area, and wherein a second plurality of EEG traces, corresponding to the second set of montages, is displayed in the first display area; and automatically generating a third set of montages based on a third plurality of contacts positioned in the vicinity of the computed vector dipole source.

Optionally, the selected at least one EEG trace corresponds to an abnormal event.

Optionally, the region of interest is specified by drawing a 3D geometrical shape around the automatically selected at least one contact, wherein at least a size of the 3D geometrical shape determines automatic selection of the first plurality of contacts.

Optionally, the first plurality of contacts is positioned within an electrode and/or across electrodes.

Optionally, the lead field matrix describes an electric potential that a unit dipole would produce at each of the first plurality of contacts.

Optionally, each of the initial vector dipole source and the computed vector dipole source is associated with a position, an orientation and an amplitude.

Optionally, the head model is generated by associating a volume conductivity of the patient's brain with the three-dimensional image of the patient's brain.

Optionally, the computed vector dipole source is determined by: generating a composite error value by comparing the plurality of expected voltages with a plurality of actual voltages at at least a portion of the first plurality of contacts within the region of interest; iteratively changing dimensions of the initial vector dipole source to generate successive vector dipole sources; and iteratively generating a successive composite error value for each of the successive vector dipole sources in a direction of decreasing composite error value until a minimum composite error is determined, wherein the computed vector dipole source corresponds to the minimum composite error.

Optionally, the computed vector dipole source is displayed in the second display area.

Optionally, the third set of montages is displayed in the second display area, wherein a third plurality of EEG traces is displayed in the first display area, and wherein the third plurality of EEG traces correspond to the third set of montages.

In still some other embodiments, the present specification is also directed to a computer-implemented method of enabling a user to generate one or more montages using a computing device, wherein the computing device comprises at least one processor configured to execute programmatic instructions and a non-transient memory adapted to store the programmatic instructions, comprising: generating a graphical user interface, wherein the graphical user interface comprises at least a first display area and a second display area, wherein the first display area is separate and distinct from the second display area, and wherein the first and second display areas are positioned side-by-side; acquiring, using the computing device, post-surgical data, wherein the post-surgical data comprises a three-dimensional image of a patient's brain and comprises data indicative of a visual representation of one or more electrodes surgically placed in the patient's brain, wherein each of the one or more electrodes has associated one or more contacts, and wherein the three-dimensional image is co-registered with a three-dimensional coordinate system; acquiring, using the computing device, EEG data of the patient; displaying, simultaneously, the EEG data in the first display area and the post-surgical data in the second display area of the graphical user interface, wherein the EEG data is displayed as a first plurality of EEG traces; automatically visually associating the visual representation of each of the one or more electrodes and associated one or more contacts in the post-surgical data with a corresponding one of the first plurality of EEG traces; selecting, by the user, at least one EEG trace of the first plurality of EEG traces thereby causing at least one contact to be automatically selected in the second display area, wherein said automatically selected at least contact is associated with said selected at least one EEG trace; specifying, by the user, a region of interest around the automatically selected at least one contact thereby causing a first plurality of contacts, within the region of interest, to be automatically selected; automatically generating a first set of montages based on the first plurality of contacts; automatically computing a lead field matrix using the head model; automatically generating an initial vector dipole source using data indicative of a first plurality of voltages associated with said at least one EEG trace of the first plurality of EEG traces; automatically determining a plurality of expected voltages generated by the initial vector dipole source at at least a portion of the first plurality of contacts within the region of interest; automatically computing a computed dipole source based on said plurality of expected voltages; and automatically generating a second set of montages based on a second plurality of contacts positioned in the vicinity of the computed vector dipole source.

Optionally, the computer-implemented method further comprises: automatically generating a plurality of virtual contacts based on the computed vector dipole source, wherein the plurality of virtual contacts are displayed in the second display area; and automatically generating a third set of montages based on the plurality of virtual contacts, wherein the third set of montages is displayed in the second display area, and wherein a third plurality of EEG traces, corresponding to the third set of montages, is displayed in the first display area.

The aforementioned and other embodiments of the present specification shall be described in greater depth in the drawings and detailed description provided below.

### BRIEF DESCRIPTION OF THE DRAWINGS

The accompanying drawings illustrate various embodiments of systems, methods, and embodiments of various other aspects of the disclosure. Any person with ordinary skill in the art will appreciate that the illustrated element boundaries (e.g. boxes, groups of boxes, or other shapes) in the figures represent one example of the boundaries. It may be that in some examples one element may be designed as multiple elements or that multiple elements may be designed as one element. In some examples, an element shown as an internal component of one element may be implemented as an external component in another and vice versa. Furthermore, elements may not be drawn to scale. Non-limiting and non-exhaustive descriptions are described with reference to the following drawings. The components in the figures are not necessarily to scale, emphasis instead being placed upon illustrating principles.
FIG. 1 is a block diagram illustrating an integrated multi-modality system, in accordance with some embodiments of the present specification;
FIG. 2 is a flowchart illustrating multiple steps in an integrated multi-modality exam workflow, focused on localizing neural sources of an abnormal or seizure event, in accordance with some embodiments of the present specification;
FIG. 3A illustrates a first unified viewing environment that displays EEG traces alongside a visual representation of the corresponding electrodes and contacts within a 3D model of a patient's brain, in accordance with some embodiments of the present specification;
FIG. 3B presents an alternate view of the first unified viewing environment, shown in FIG. 3A, allowing a clinician to visualize the association between EEG and imaging data within the patient's 3D brain model and to select one or more contacts of interest, in accordance with some embodiments of the present specification;
FIG. 3C depicts an additional view of the first unified viewing environment, shown in FIG. 3A, enabling the clinician to visualize the association between EEG and imaging data within the patient's 3D brain model and to select one or more contacts of interest, in accordance with some embodiments of the present specification;
FIG. 3D presents a second unified viewing environment that illustrates automatic selection of at least one electrode contact within a patient's 3D brain model based on identification and selection of at least one EEG trace related to an abnormal event, in accordance with some embodiments of the present specification;
FIG. 3E illustrates a third unified viewing environment that displays an anatomical atlas within a patient's 3D brain model, in accordance with some embodiments of the present specification;
FIG. 3F illustrates a fourth unified viewing environment that displays an initial vector dipole source within a patient's 3D brain model, in accordance with some embodiments of the present specification;
FIG. 3G presents a portion of the fourth unified viewing environment, shown in FIG. 3F, that displays multiple interim dipoles and an estimated best-fitting vector dipole source within the patient's 3D brain model, in accordance with some embodiments of the present specification;
FIG. 3H presents the portion of the fourth unified viewing environment, shown un FIG. 3G, that displays a plurality of virtual contacts within the patient's 3D brain model, in accordance with some embodiments of the present specification; and
FIG. 4 illustrates exemplary montages that are generated based on a standard 10-20 system of placing electrode contacts on the scalp of a patient, in accordance with some embodiments of the present specification.

### DETAILED DESCRIPTION

The present specification describes systems and methods for algorithmically generating virtual contacts to assist in identifying the neural source of abnormal or seizure activity, through visualization of EEG data. In some embodiments, a module is configured to use three-dimensional region of interest (3D ROI) montaging to analyze EEG traces and identify the largest recorded voltages associated with a suspected spike event or event of interest. Using this information, the system is configured to model a best-fit or computed vector dipole source within the brain. The modeled dipole is then used either to select nearby electrode contacts for montage creation or generate virtual electrodes that can be more evenly distributed around the vector dipole source to facilitate montage development.

In embodiments, the systems and methods of the present specification enable an automatic visual association of the visual representation of each of the one or more electrodes and associated one or more contacts in the post-surgical data with a corresponding at least one EEG trace using several methods. These methods enable real-time correlation between electrophysiological signals and neuroanatomical structures while minimizing manual input. In an embodiment, metadata matching (contact IDs, spatial coordinates), in which electrode metadata is matched with EEG channel labels using predefined configurations is employed. This may include manufacturer-specific electrode profiles with dimensional specifications and anatomical tagging data. In some embodiments, 3D spatial alignment methods are employed, in which virtual electrodes from pre-surgical plans are algorithmically aligned with CT-detected contacts in the 3D brain model. Automatic refinement may be used to adjust virtual electrode positions to match CT artifacts, while manual calibration allows for interactive adjustments. In some embodiments, bi-directional GUI interaction is used, by selecting an EEG trace which results in the highlighting of its associated contact in the 3D model and vice versa. In embodiments, multi-trace selections trigger collective highlighting of all related contacts across electrodes. In embodiments, the methods also include ROI-based montage generation in which a 3D geometric shape (e.g., sphere) drawn around a contact auto-selects all included contacts, dynamically updating EEG traces linked to the region. In still further embodiments, methods may include anatomical atlas integration in which predefined anatomical regions (color-coded in the 3D model), when selected, result in the automatic highlighting of contacts and their EEG traces. Still further, surgical data co-registration techniques are employed in which pre-surgical MRI trajectories and post-surgical CT electrode positions are co-registered in a unified coordinate system to resolve EEG-anatomy relationships. The systems and methods of the present specification also support differential visualization tools that enable the display of deviations between planned and actual electrode placements, with thresholds for highlighting clinically relevant positional offsets.

In embodiments, the systems and methods of the present specification automatically associated selected contacts with their corresponding EEG traces through specific data structure linkages. For example, a contact ID may be directly linked to predefined EEG channel labels in metadata configurations. Further, manufacturer-specific electrode profiles (such as dimensions, contact spacing) may be mapped to montage channel groupings in the system's database. In addition, post-surgical CT-derived 3D coordinates of contacts are algorithmically aligned with pre-surgical virtual electrode trajectories, linking spatial positions to EEG trace indices. Further, an automatic refinement of virtual electrode positions may result in the dynamic update of associations between CT-detected contacts and EEG traces in real-time. Still further, selecting an EEG trace triggers a highlighting of the associated contact in the 3D model via trace-to-contact index pointers. In addition, the selection of multiple EEG traces generates a list of contact indices that may be stored for simultaneous highlighting. In addition, a 3D geometric shape (e.g., sphere) defined by center coordinates and radius filters contacts via spatial inclusion checks, dynamically updating linked EEG traces. Further, contacts with an ROI are stored in a subset array that is linked to montage-specific EEG trace groups. Predefined anatomical regions (color-coded in the 3D model) are stored as metadata tags for contacts, enabling auto-selection of traces when regions are chosen and neural network associations between contacts and functional areas influence trace display order in montages. In addition, transformation matrices co-register pre-surgical MRI trajectories with post-surgical CT contacts, linking both to EEG channels through shared coordinate references. Further, positional offsets between planned/actual contacts are stored as threshold parameters that trigger visual alerts in associated EEG traces. In addition, event-driven associations include logging of timestamps of detected abnormal events with corresponding contact indices, creating temporal-spatial links to EEG traces. Further, computed dipole coordinates (x, y, z, orientation, amplitude) generate virtual contact grids stored in matrices linked to synthetic EEG channels. These associations are implemented through structured data objects including: electrode metadata Tables (contact IDs, spatial coordinates, anatomical tags); montage configuration files (channel labels, reference schemes); 3D coordinate arrays (pre/post-surgical spatial data); event-contact mapping Arrays (GUI interaction logs); and/or ROI parameter sets (geometric definitions, inclusion lists).

The present specification is directed towards multiple embodiments. The following disclosure is provided in order to enable a person having ordinary skill in the art to practice the invention. Language used in this specification should not be interpreted as a general disavowal of any one specific embodiment or used to limit the claims beyond the meaning of the terms used therein. The general principles defined herein may be applied to other embodiments and applications without departing from the spirit and scope of the invention. Also, the terminology and phraseology used is for the purpose of describing exemplary embodiments and should not be considered limiting. Thus, the present invention is to be accorded the widest scope encompassing numerous alternatives, modifications and equivalents consistent with the principles and features disclosed. For purpose of clarity, details relating to technical material that is known in the technical fields related to the invention have not been described in detail so as not to unnecessarily obscure the present invention.

In various embodiments, a computing device includes an input/output controller, at least one communications interface and system memory. The system memory includes at least one random access memory (RAM) and at least one read-only memory (ROM). These elements are in communication with a central processing unit (CPU) to enable operation of the computing device. In various embodiments, the computing device may be a conventional standalone computer or alternatively, the functions of the computing device may be distributed across multiple computer systems and architectures.

In some embodiments, execution of a plurality of sequences of programmatic instructions or code enable or cause the CPU of the computing device to perform various functions and processes. In alternate embodiments, hard-wired circuitry may be used in place of, or in combination with, software instructions for implementation of the processes of systems and methods described in this application. Thus, the systems and methods described are not limited to any specific combination of hardware and software.

The term "module" used in this disclosure may refer to computer logic utilized to provide a desired functionality, service or operation by programming or controlling a general-purpose processor. Stated differently, in some embodiments, a module, application or engine implements a plurality of instructions or programmatic code to cause a general-purpose processor to perform one or more functions. In various embodiments, a module, application or engine can be implemented in hardware, firmware, software or any combination thereof. The module, application or engine may be interchangeably used with unit, logic, logical block, component, or circuit, for example. The module, application or engine may be the minimum unit, or part thereof, which performs one or more particular functions.

The term "montage" used in this disclosure may refer to a logical arrangement of channels - each representing a comparison between two EEG amplifier inputs - that is used to convey information about electrical signals in the brain. As used in this specification, a montage can be bipolar or differential, and it may be generated from selected physical contacts or virtual contacts near a modeled dipole source.

In embodiments of the present specification, an "EEG trace" is the waveform of a montage channel created by taking the mathematical difference of two input waveforms, so a montage is essentially the channel layout that produces those traces. Further, in embodiments, montages may be created to emphasize local signal gradients, minimize noise, and help localize an abnormal event or neural source.

As used in this specification, "post-surgical data" is used to refer to post-surgical brain data that includes a three-dimensional image of the patient's brain and a visual representation of implanted electrodes with their associated contacts. In some embodiments, the present specification used post-surgical data as the imaging side of a side-by-side interface, where it is displayed together with EEG traces and used to automatically link traces to contacts, define a region of interest, and generate montages.

As used throughout this specification, "head model" refers to a patient-specific 3D brain-based model that represents the electrical properties of the brain, skull, and cerebrospinal fluid so the system can simulate how brain signals propagate to the electrodes. In embodiments, it is used as the basis for computing the lead field matrix, which maps a unit dipole source in the brain to the voltages expected at the electrode contacts. In some embodiments, the head model may be generated by associating a volume conductivity with the patient's three-dimensional brain image, so it functions as the forward-modeling representation used for dipole localization and montage optimization.

In some embodiments, the term "region of interest" refers to a user-specified area in the 3D brain model, typically drawn as a 3D geometrical shape such as sphere around a selected contact, that automatically includes nearby electrode contacts for focused analysis. Throughout the specification, it may be used to select a first plurality of contacts within it, generate targeted montages, and refine dipole modeling by limiting computations to contacts inside the ROI.

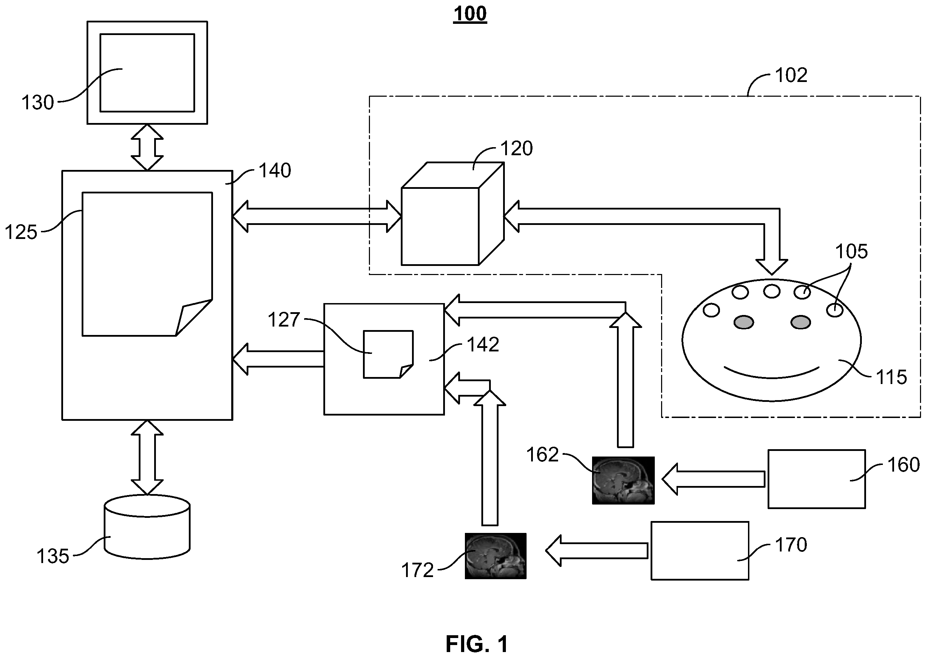

The term "average reference", as used in this disclosure, may denote a referencing method in which, for a defined region surrounding a selected contact, the electrical activity from all contacts within that region is averaged to serve as a reference for other contacts within the same space. The region may take the form of a sphere, cube, another standard geometric shape, or a custom-defined volume of interest. In some embodiments, a module (such as module 125 described with reference to FIG. 1 below) is configured to automatically select which electrodes are included in the average, based on their signal characteristics or content. If an electrode exhibits excessive activity, its signal may be excluded from the average.

As used throughout the specification, a contact is in a region of interest if the pixels that visually define the contact are within the visual area circumscribed by the region of interest. In embodiments, the percentage of contacts that are selected within a region of interest is preferentially 100% of the contacts, but may be a smaller number or select percentage, such 60% -95% or any numerical increment therein.

As used in this specification, the term "lead field matrix" refers to a mathematical construct computed from the head model that represents the electric potential generated at each electrode contact by a unit dipole at a specific position and orientation in the brain.

The term "inverse solution" used in this disclosure, in the context of electrophysiology or neuroimaging, may refer to a mathematical method used to infer the source(s) of electrical activity (such as dipoles) inside a body (like the brain) based on measurements recorded on the surface of the brain (e.g., EEG, MEG, or scalp voltages).

As used in this specification, the term "initial vector dipole source" refers to an automatically generated preliminary estimate of a brain dipole (positioned in 3D space with orientation and amplitude) that represents the likely origin of an abnormal EEG event like a spike. In embodiments, it may be created using data from the highest-amplitude voltages in a selected EEG trace (or traces) within a region of interest, serving as the starting point for iterative optimization toward a best-fit computed vector dipole source. In embodiments, the initial vector dipole source includes six parameters: x, y, z position; yaw and pitch orientation; and amplitude, used with the lead field matrix to predict expected voltages at contacts for error minimization.

In embodiments, the term "computed vector dipole source" or "computed dipole source" refers to the optimized, best-fitting estimate of a brain dipole's location, orientation, and amplitude that minimizes the error between predicted and actual EEG voltages recorded at electrode contacts. In embodiments of the specification, it is automatically derived from an initial vector dipole source by iteratively adjusting its six parameters (x, y, z position; yaw, pitch orientation; amplitude) to achieve the minimum composite error value.

In embodiments, as used in this specification, successive vector dipole sources refer to intermediate candidate dipole models generated during an iterative optimization process to refine an initial vector dipole source into the final computed vector dipole source. In embodiments, each successive source is created by incrementally changing the six dimensions (position x/y/z, orientation yaw/pitch, amplitude) of the previous dipole, with a successive composite error value calculated for each to measure fit between predicted and actual EEG voltages at contacts. In embodiments of the present specification, the process continues in the direction of decreasing error until a minimum composite error is reached, at which point the corresponding successive dipole becomes the computed dipole source representing the neural origin of the abnormal event. This optimization uses the lead field matrix for forward predictions within the region of interest.

In embodiments, "virtual contacts" are algorithmically generated simulated electrode contacts placed around a computed dipole source in the 3D brain model to improve EEG montage visualization and neural source localization (and, in particular neural source localization of abnormal events such as spikes). In the specification, they are automatically created based on the optimized computed vector dipole source and displayed in the second display area of the GUI alongside physical contacts.

In the description and claims of the application, each of the words "comprise", "include", "have", "contain", and forms thereof, are not necessarily limited to members in a list with which the words may be associated. Thus, they are intended to be equivalent in meaning and be open-ended in that an item or items following any one of these words is not meant to be an exhaustive listing of such item or items, or meant to be limited to only the listed item or items. It should be noted herein that any feature or component described in association with a specific embodiment may be used and implemented with any other embodiment unless clearly indicated otherwise.

It should also be noted that as used herein and in the appended claims, the singular forms "a," "an," and "the" include plural references unless the context dictates otherwise. Although any systems and methods similar or equivalent to those described herein can be used in the practice or testing of embodiments of the present disclosure, the preferred, systems and methods are now described.

### Overview

FIG. 1 is a block diagram illustrating an integrated multi-modality system, in accordance with some embodiments of the present specification. In various embodiments, system 100 offers integration, visualization, manipulation, and analysis of electroencephalography (EEG) and medical imaging data. In some configurations, system 100 includes a first modality - an EEG system 102 - configured to detect, diagnose, or predict neurological events based on EEG signals. As illustrated in FIG. 1, a plurality of EEG sensors or electrodes 105 are either implanted in the patient's brain or positioned on a tissue layer such as the scalp of the patient 115. The plurality of electrodes 105 are in data communication with a multi-channel amplifier 120, which, in turn is in data communication with a first computing device 140. The first computing device 140, in turn, is in data communication with both a display unit 130 and at least one database 135.

In various embodiments, the plurality of electrodes 105 include grid, strip and/or depth electrodes where each electrode may have multiple contacts. The plurality of electrodes 105 record electrical signals (EEG signals) from the patient's brain and communicate the analog signals using a first communication link to the multi-channel amplifier 120 that amplifies the signals, converts the signals from an analog EEG data set to a digital EEG data set, and communicates the resultant digital EEG data to the first computing device 140 over a second communication link.

In some embodiments, the system 100 incorporates a second modality featuring a first imaging system 160 that generates patient-specific first image data 162 and transmits the data 162 to a second computing device 142 via a third communication link. Additionally, system 100 may include a third modality featuring a second medical imaging system 170 that generates patient-specific second image data 172 and transmits the data 172 to the same second computing device 142 over a fourth communication link.

In various embodiments, the image data generated by the first imaging system 160 and the second imaging system 170 may be derived from a range of imaging modalities, including but not limited to Magnetic Resonance Imaging (MRI), Computed Tomography (CT), Single Proton Emission Computed Tomography (SPECT), or Positron Emission Tomography (PET) imaging. In an embodiment, the second medical imaging system is an ionizing radiation-based medical imaging system. In various embodiments, each of the plurality of imaging modalities provide image data consisting of a series of images representing information in 3D space.

The second computing device 142 includes a third-party surgical planning software or applications 127 such as ROSA (Robotic Stereotactic Assistance), which are utilized to generate processed pre- and post-surgical data. In accordance with some aspects of the present specification, a clinician can import this processed data from the second computing device 142 into the first computing device 140 via a fifth communication link. In embodiments, the first, second, third, fourth and fifth communication links may be dynamic, identical or distinct, and can be implemented using either wired or wireless connections.

Each of the first computing device 140 and second computing device 142 is equipped with an input/output controller, at least one communications interface and system memory that includes both random access memory (RAM) and read-only memory (ROM). These components interface and are in communication with a central processing unit (CPU) to support the operation of each device. In various embodiments, each of the first computing device 140 and second computing device 142 may be implemented as a conventional standalone computer, or their functions can be distributed across multiple computer systems and architectures. For example, in a distributed architecture for the first computing device 140, the database 135 and processing circuitry may be located in separate units or locations, with some units dedicated to primary processing tasks and containing, at minimum, a general controller or a processing circuitry along with system memory.

### Pre-Surgical Data and Post-Surgical Data

### Pre-Surgical Data

As mentioned earlier, the second computing device 142 includes a third-party surgical planning software or application 127 that is configured to process and support co-registration of the patient-specific first image data 162 and second image data 172.

During a pre-surgical planning stage, the patient is initially imaged using the second modality to generate the first image data 162. In some embodiments, the second modality is a magnetic resonance imaging (MRI) system, and the resulting first image data 162 consists of MRI image data that provides three-dimensional spatial information.

The second computing device 142 receives MRI image data via the third communication link and processes it using the third-party surgical planning software 127 (e.g. ROSA). The key processing steps include, but are not limited to, removing background noise, optimizing images by adjusting brightness/contrast levels, automated or manual brain isolation (for example by selecting, either manually or by using an automated tool, the area of the brain and removing extracorporeal data), co-registration via spatial adjustments (adjusting offsets/rotations), and/or alignment with a predefined or standard three-dimensional coordinate system. The processing steps generate processed pre-surgery image data 162 representing an MRI-derived 3D brain model such as a Cortical Surface Projection model mapping the brain's outer surface geometry.

The third-party surgical planning software 127 uses the MRI-based 3D brain model to simulate optimal electrode configurations or placement of virtual electrodes, generating a detailed surgical plan. The surgical plan provides a baseline/goal visual representation of electrode placements or trajectories including a trajectory list, where the list includes data indicative of dimensions of each electrode (length/diameter) and the spatial start/end coordinates for each electrode. Thus, the virtual electrodes are projected onto the 3D Cortical Surface Projection model to simulate/view where the electrodes should be placed, enabling visualization of the virtual electrodes.

Typically, virtual electrode types are selected from a manufacturer-specific library of electrode profiles. The selected electrodes are manually positioned on the MRI-based 3D brain model. Electrode profiles include information such as, but not limited to, electrode types (grid, depth, and other intracranial devices), physical specifications of the electrode (including dimensions, contact counts, and material composition), and optional metadata (date of first use, sterilization details, manufacturing lot). The electrode profiles enable accurate 3D model integration by providing scalable electrode rendering based on physical measurements, anatomical tagging (positioning within white/gray matter), and/or neural network associations for functional mapping. Alternatively, ROSA (Robotic Stereotactic Assistance) trajectories, which are robot-assisted electrode placement trajectories, may be imported into the third-party surgical planning software 127. The imported virtual electrodes may then be manually aligned or manipulated to match these trajectories.

Thus, the third-party surgical planning software 127 is used to generate pre-surgical data that includes a) processed and co-registered pre-surgery first image data 162, representing an MRI-based 3D brain model, and b) a surgical plan, which provides a baseline/goal visual representation of the intended electrode placements or trajectories, along with a trajectory list and electrode profiles. In various embodiments, the first image data 162 may be derived from MRI, CT, SPECT or PET scans, resulting in a 3D brain model based on any of these imaging modalities.

### Post-surgical data

Following the surgical implantation or placement of electrodes in the patient's brain, a precise patient-specific 3D anatomical model is generated, visually depicting the location, position, orientation, and dimensions of the implanted electrodes. This is accomplished by using the third modality to generate the second image data 172. In some embodiments, the third modality is a CT system, and the second image data 172 consists of a series of CT images that provide three-dimensional spatial information.

The second computing device 142 receives CT image data and processes it using the third-party surgical planning software 127 (e.g. ROSA).

The key processing steps include, but are not limited to, removing background noise, optimizing images by adjusting brightness/contrast levels, automated or manual brain isolation (for example by selecting, either manually or by using an automated tool, the area of the brain and removing extracorporeal data), co-registration via spatial adjustments (adjusting offsets/rotations), and/or alignment with the same predefined or standard three-dimensional coordinate system as first image data 162. The processing steps generate processed post-surgery image data 172 representing a CT-derived 3D brain model of the patient. In various embodiments, the second image data 172 is at least one of MRI, CT, SPECT or PET based 3D brain model.

### Multi- modality exam module or engine 125

In accordance with aspects of the present specification, the first computing device 140 includes a multi-modality exam module or engine 125. Module or engine 125 executes a set of programmatic instructions that enable it to implement an integrated multi-modality exam workflow. As a result, the module 125 can record, display, analyze, and review data from the first exam modality 102, the second exam modality 160, and the third exam modality 170, presenting this information as unified, multi-modal data.

Any steps not expressly tied to a specific purpose-built hardware embodiment may be performed by a general-purpose computing device executing appropriate programmatic code. In some embodiments, the execution of programmatic instruction sequences enables or causes the CPU to perform various functions and processes. In alternate embodiments, hard-wired circuitry may be used either instead of, or alongside, software instructions to implement the processes of the systems and the methods described in this application. Therefore, the systems and methods described herein are not restricted to any particular combination of hardware and software.

### Integrated Multi-Modality Exam Workflow

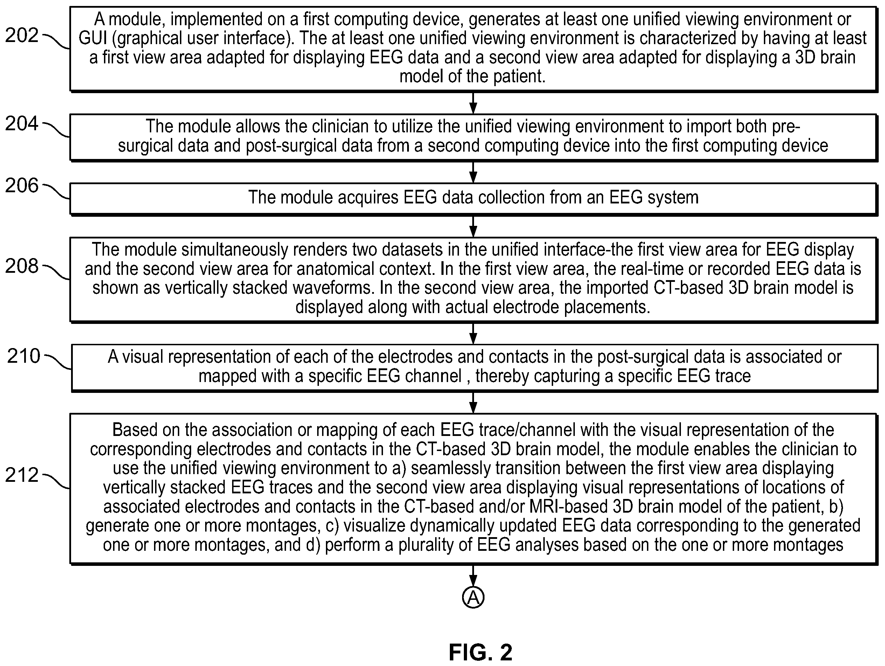

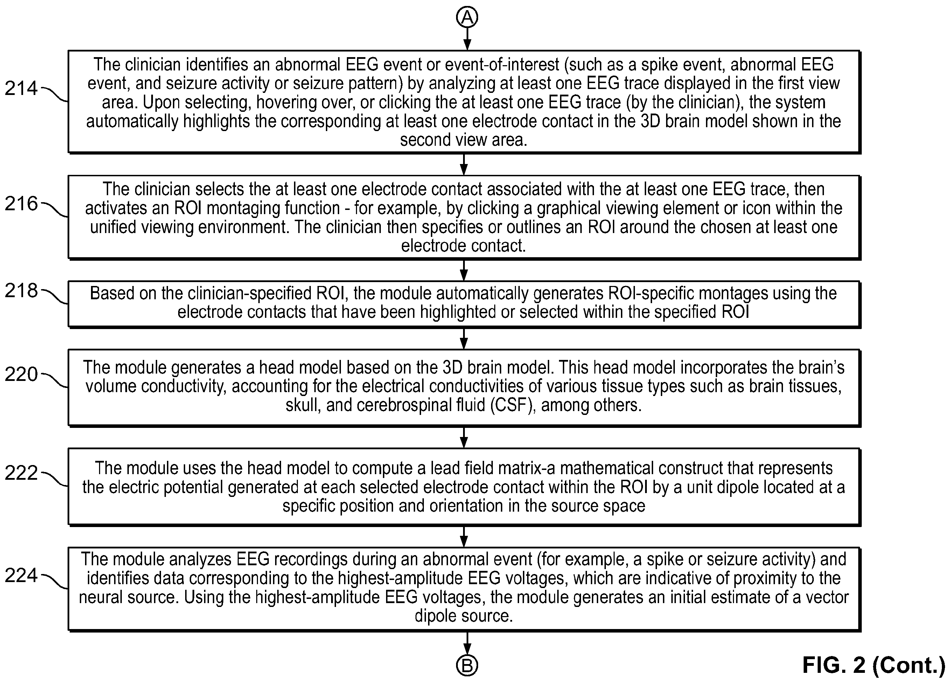

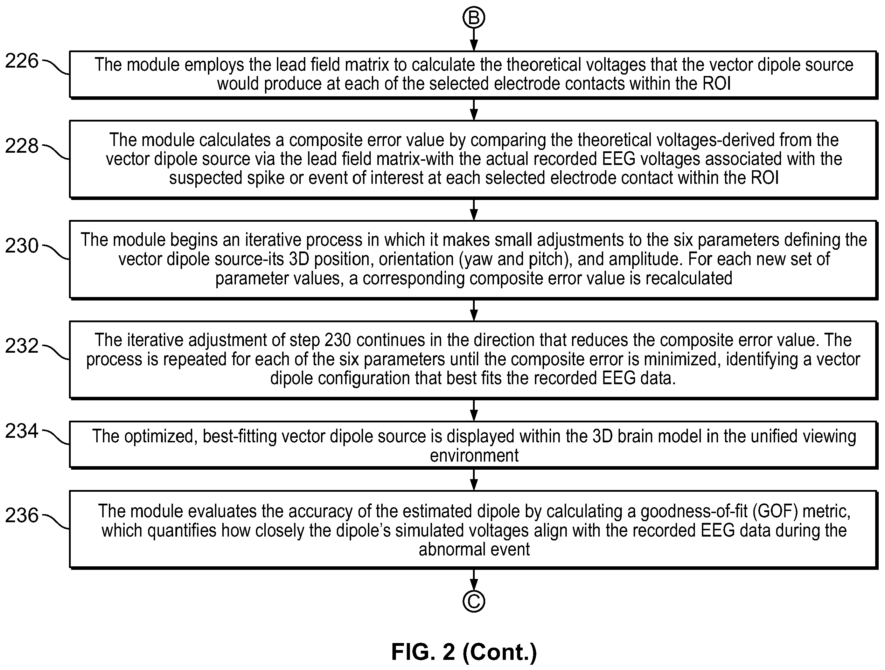

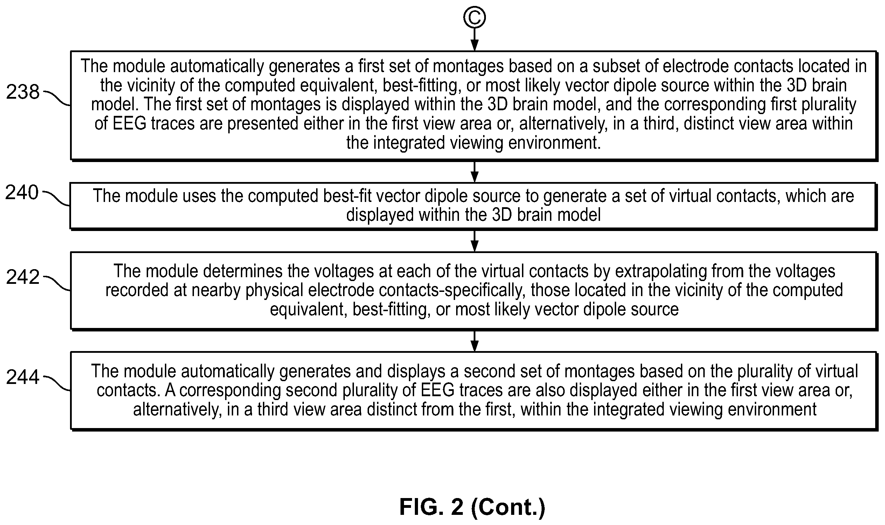

FIG. 2 is a flowchart illustrating multiple steps in an integrated multi-modality exam workflow, focused on localizing neural sources of an abnormal or seizure event, in accordance with some embodiments of the present specification. In embodiments, the module 125 is configured to enable execution of the integrated multi-modality exam workflow.

Referring now to FIGS. 1 and 2, at step 202, the module 125 generates at least one unified viewing environment or GUI (graphical user interface) characterized by at least a first view area adapted for displaying EEG data and a second view area adapted for displaying a 3D brain model of the patient. In some embodiments, the first view area is separate and distinct from the second view area (that is, they do not overlap). In some embodiments, the first view area and second view area are positioned side-by-side (that is, adjacent to each other). In some embodiments, the at least one unified viewing environment also includes a third view area adapted for displaying a list of electrode contacts, and optionally, metadata associated with each contact (impedance, signal quality).

At step 204, the pre-configured module 125 allows the clinician to utilize the unified viewing environment to import both pre-surgical data and post-surgical data from the second computing device 142 into the first computing device 140. In some embodiments, the pre-surgical data represents a) processed and co-registered pre-surgery first image data 162, representing an MRI-based 3D brain model of the patient, and b) a surgical plan, a visual baseline or target for electrode placements or trajectories, along with a trajectory list and electrode profiles. In some embodiments, the post-surgical data consists of processed and co-registered post-surgery second image data 172, which corresponds to a CT-based 3D brain model of the patient.

The system of the present invention is advantageous in that the clinician is not required to manually pre-process or co-register the first image data 162, simulate pre-surgical electrode trajectories, or pre-process and co-register the second image data 172 within the unified viewing environment. Instead, with a single click, the clinician can import both pre- and post-surgical data directly from a third-party surgical planning software into the unified interface. This streamlined import process eliminates the need for additional manual steps, integrating with the standard clinical workflow and avoiding the time-consuming and error-prone task of manually co-registering MRI and CT-based 3D brain models or planning electrode trajectories.

Thus, the module 125 allows clinicians to import pre- and post-surgical data with no additional training required beyond the act of importing external files. In particular, the clinician does not need advanced knowledge or expertise in brain physiology mapping for this feature. As a result, "normal" epileptologists and technicians can perform the import, enhancing their clinical experience. In addition, the clinician is not required to carry out complicated co-registration or error analysis for the pre- and post-surgical data.

At step 206, the module 125 acquires EEG data collected from system 102. In embodiments, all electrode contacts are physically connected to inputs of amplifier 120 and one or more montages are configured (channel groupings) to isolate clinically relevant EEG signals. The amplifier 120 converts analog EEG signals to digital EEG signals for analysis. In some embodiments, the one or more montages are standard bipolar or referential. These may be automatically generated based on: referencing each contact to its adjacent contact on each electrode (bipolar), referencing each contact to a single specific contact (external reference), or referencing each contact to a common average (also referred to as 'average reference').

At step 208, the module 125 simultaneously renders two datasets in the unified viewing environment or GUI - the first view area for EEG display and the second view area for anatomical context. In the first view area, the real-time or recorded EEG data is shown as vertically stacked waveforms. In the second view area, the imported CT-based 3D brain model is displayed along with actual electrode placements. This highlights the spatial relationship between electrodes and brain structures.

At step 210, a visual representation of each of the electrodes and their contacts in the post-surgical data is associated or mapped with a specific EEG channel, thereby defining a specific EEG trace. This association or mapping may be achieved manually or automatically. The clinician can select and track the electrode contacts to stimulate and/or use as a reference and visually see which traces correspond to portions of the patient's anatomy. To enable the association, the data from each EEG trace/channel is correlated with the visual representation of a contact in the CT-based 3D brain model. The automatic association method uses electrode metadata (contact IDs and spatial coordinates) and matches anatomy with EEG channel labels via predefined configurations.

### Electrodes and contacts association or mapping with specific EEG traces

In some embodiments, virtual electrodes and contacts are selected from a library of manufacturer-specific electrode profiles that include dimensional specifications (length, contact spacing), material properties, and other metadata. The electrodes are automatically mapped to the planned pre-surgical imported trajectories (from the pre-surgical data) within the CT-based 3D brain model.

In some embodiments, module 125 enables alignment of virtual electrodes/contacts with their CT-visible counterparts in the 3D brain model through automatic refinement or manual calibration. The automatic refinement uses algorithms to adjust virtual electrode positions to match CT-detected artifacts. The manual calibration function affords clinicians the ability to interactively adjust placements via drag-and-drop or coordinate entry.

It should be appreciated that the CT-based 3D brain model allows clinicians to visualize surgically implanted electrode contacts within a patient's brain. The module 125 uses preloaded data on electrode specifications, including type and geometric design, to map CT scan pixels to specific contacts, thereby precisely determining where each contact is located within the patient's brain. By analyzing dimensions of electrodes and the relative spacing or distribution of contacts along each electrode, the module 125 uses that information to distinguish between true electrode contacts and image artifacts or noise.

In some embodiments, the configured module 125 is used to directly compare planned electrode positions (the pre-surgical models, baseline/goal visual representations) to the actual implant locations (electrode placement, the post-surgical CT data). The comparison is used to generate and display differential measurements or deviations between where the electrodes were supposed to be and where they were actually placed, thus identifying placement errors for immediate intraoperative adjustments.

The post-surgical CT-based 3D brain model visualizations highlight the differences between planned electrode positions and actual electrode positions due to various surgical, mechanical, and anatomical factors, offering post-operative 3D validation. During surgery, surgeons may adjust electrode trajectories based on real-time observations, such as vascular avoidance or tissue resistance, robotic placement systems may introduce sub-millimeter inaccuracies in placement, and/or electrodes may flex or bend along their midsection due to pressure from surrounding brain tissue.

In some embodiments, the module 125 is configured to enable and display a real-time comparison of planned electrode trajectories versus actual implant paths within a unified 3D visualization interface. In some embodiments, the module 125 is configured to highlight, in the unified viewing environment, contacts that exceed or deviate from pre-defined positional offset (relative to at least one of the MRI or CT brain model) percentages such as, for example, above 1%, above 5%, above 10%, above 15%, above 20%, above 25%, above 30%, above 50%, above 75% or any increment therein. Accordingly, the differential image shows where, relative to the pre- or post-surgical image, the projected electrodes or implanted electrodes were actually placed thereby minimizing errors in EEG activity correlations with specific patient anatomical regions. In embodiments, the offsets or thresholds are customizable based on procedural requirements.

At step 212, the configured module 125 enables the clinician to use the unified viewing environment to a) seamlessly transition between the first view area displaying vertically stacked EEG traces and the second view area displaying visual representations of locations of associated electrodes and contacts in the CT-based and/or MRI-based 3D brain model of the patient, b) create one or more montages, c) visualize dynamically updated EEG data corresponding to the created one or more montages, that are linked to anatomical positions in the 3D model, and d) perform a plurality of EEG analyses (such as spectral analysis, coherence mapping, or event detection) based on the generated one or more montages.

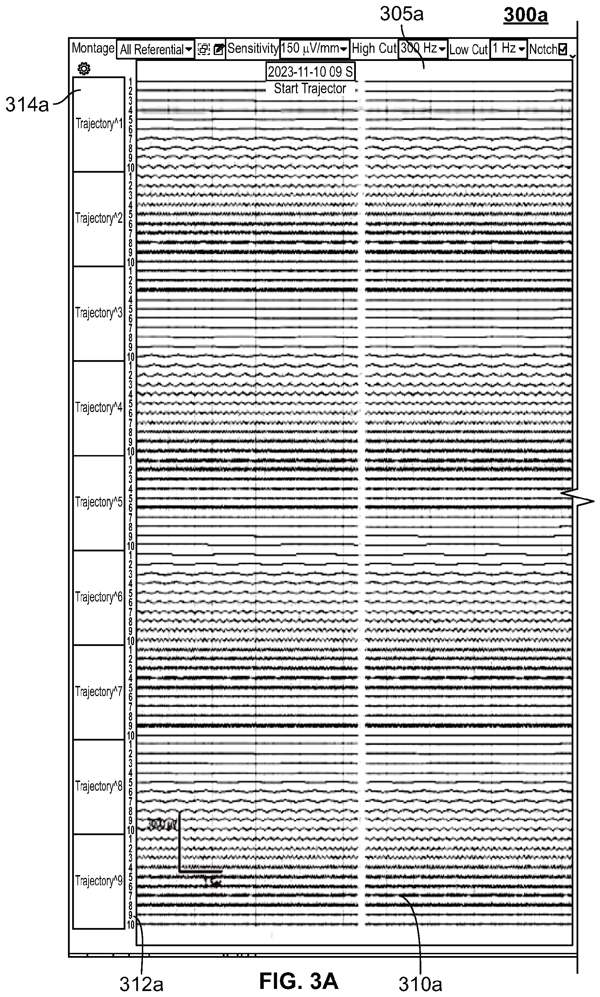

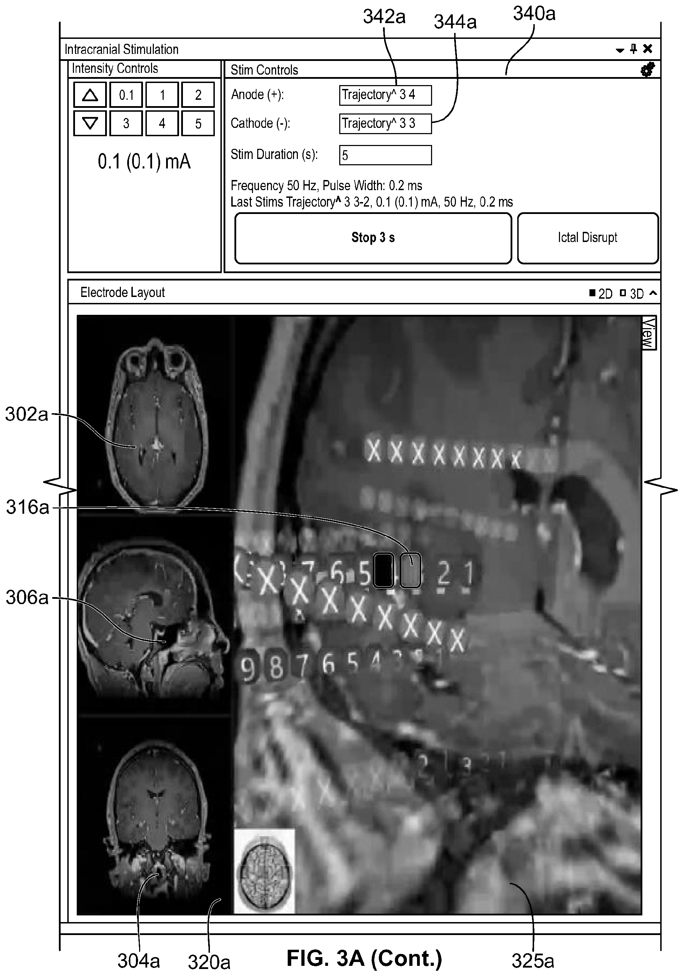

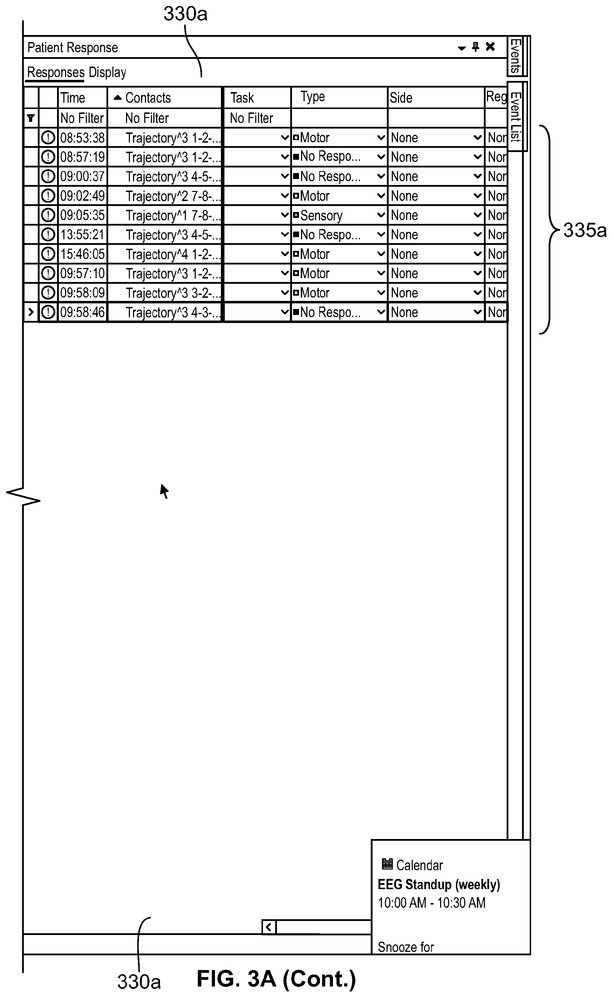

FIG. 3A illustrates a first unified viewing environment that displays EEG traces alongside a visual representation of the corresponding electrodes and contacts within a 3D model of a patient's brain, in accordance with some embodiments of the present specification. In embodiments, the module 125 is configured to generate the unified viewing environment 300a that provides multimodal data visualization in a single platform, enabling a plurality of functionalities for use by a clinician. The unified viewing environment 300a comprises a first view area 305a to display a plurality of vertically stacked EEG traces 310a along with corresponding electrode contacts 312a and electrodes/trajectories 316a, whereby the electrode trajectory name is represented in area 314a. In embodiments, the unified viewing environment 300a comprises a second view area 320a for displaying visual representations of a plurality of electrodes and contacts within a 3D brain model 325a. As described above, the 3D brain model can be generated either from a CT scan co-registered with the actual positions of surgically implanted electrodes or from and MRI scan co-registered with the planned electrode trajectories for the patient. In some embodiments, the unified viewing environment 300a includes a third view area 330a that is used to display a list 335a of the plurality of electrode contacts and associated stimulation response characteristics of the patient. In some embodiments, the unified viewing environment 300a includes a fourth view area 340a for displaying a first drop-down list 342a that is used to select and designate an electrode contact from the plurality of electrode contacts as an anode and a second drop-down list 344a that is used to select and designate an electrode contact from the plurality of electrode contacts as a cathode. The second view area 320a also displays a first image view 302a, a second image view 304a, and a third image view 306a of the 3D brain model 325a of the patient, wherein the various numbers represent the plurality of electrode contacts.

In some embodiments, the first view area 305a is separate and distinct from the second view area 320a (that is, they do not overlap). In some embodiments, the first view area 305a and second view area 320a are positioned side-by-side (that is, adjacent to each other).

By associating each EEG trace or channel with its corresponding electrode and contact in the 3D brain model, clinicians can readily identify and visualize the anatomical origin of EEG signals. The current montage defines the relationship between electrode contacts and EEG traces, and both the montage and the 3D brain model with virtual electrodes and contacts are visible simultaneously. Selecting an electrode contact on the 3D brain model in the second view area 320a highlights its corresponding EEG trace in the first view area 305a, and selecting a specific EEG trace in the first view area 305a highlights the associated contact(s) in the 3D brain model 325a, facilitating interpretation of multimodal data.

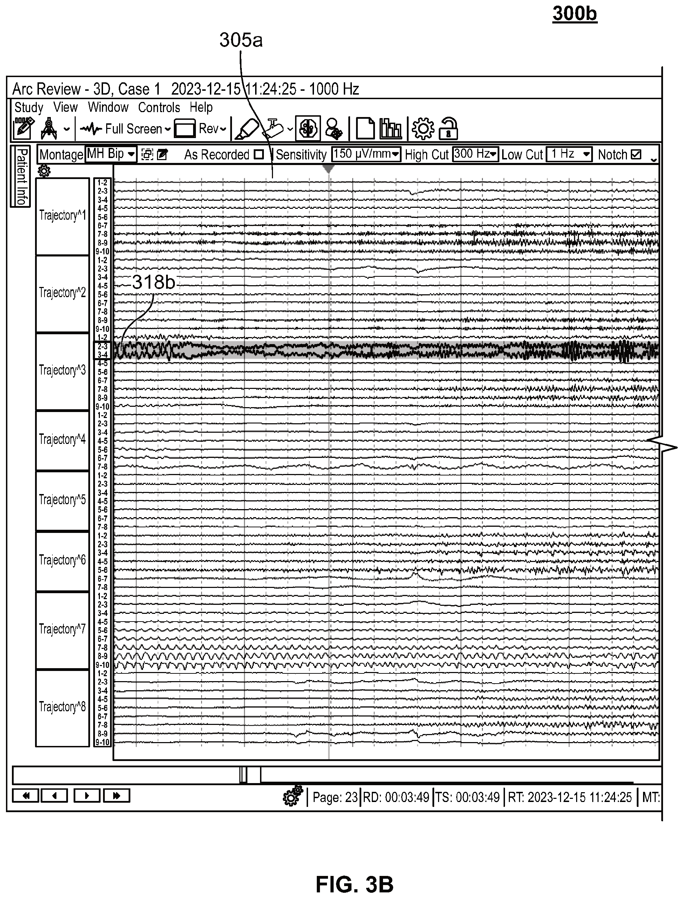

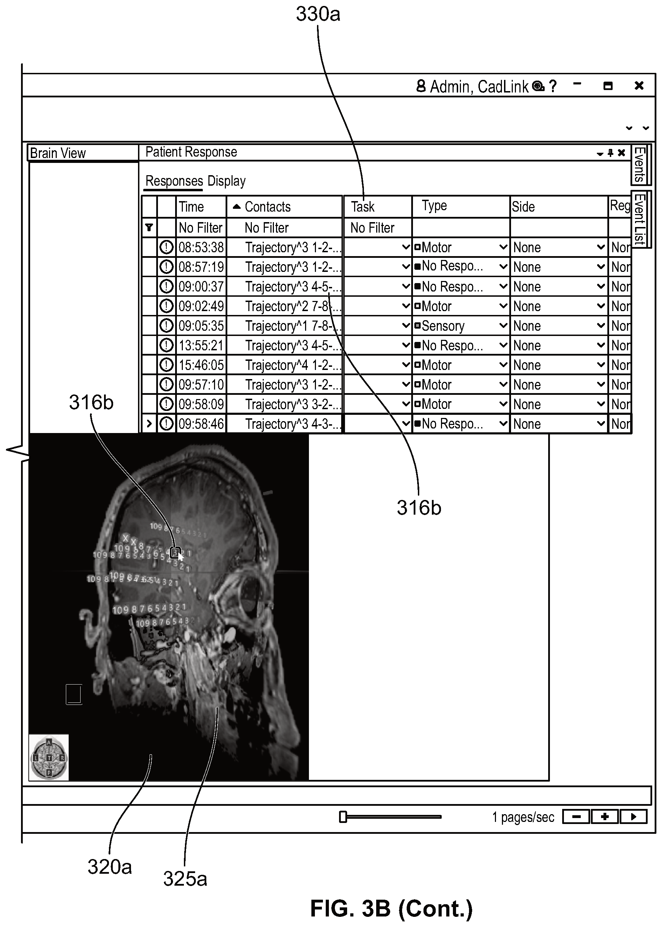

As shown in the unified viewing environment 300b of FIG. 3B, if the clinician selects (click or touch), points to, hovers over, or moves the mouse cursor over an electrode contact 316b in the 3D brain model 325a displayed in the second view area 320a, the corresponding one or more EEG trace(s) 318b, are automatically highlighted in the first view area 305a. Simultaneously, the selected associated electrode contact 316b is also automatically highlighted in the list of electrode contacts in the third view area 330a. Similarly, if the clinician selects (click or touch), hovers over, or moves a mouse cursor over an entire electrode in the 3D brain model 325a, all EEG traces associated with the contacts on that electrode are automatically highlighted in the first view area 305a. Additionally, all contacts belonging to that selected electrode are highlighted in the third view area 330a. Thus, the clinician can select an electrode contact or an entire electrode.

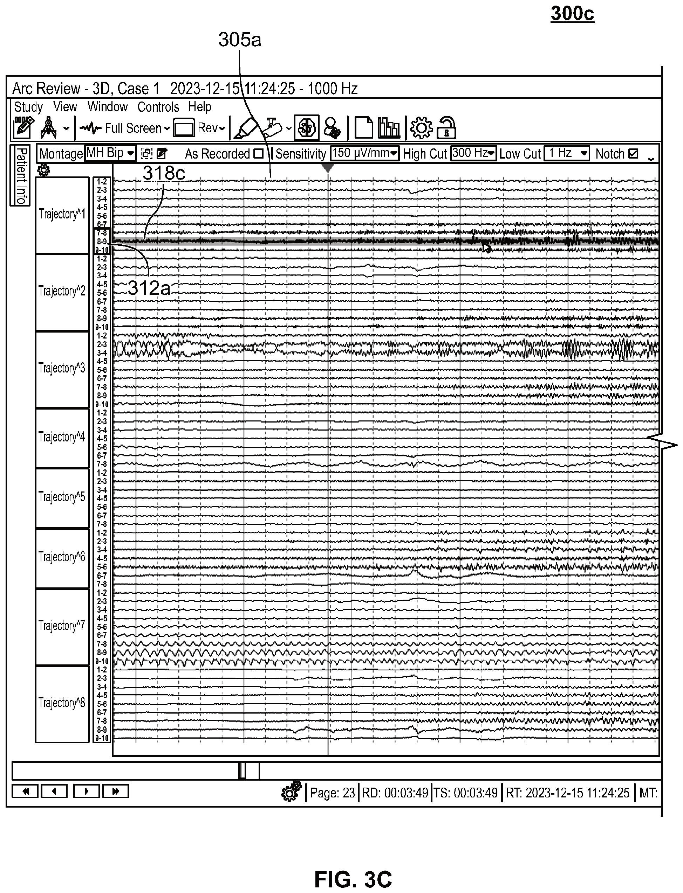

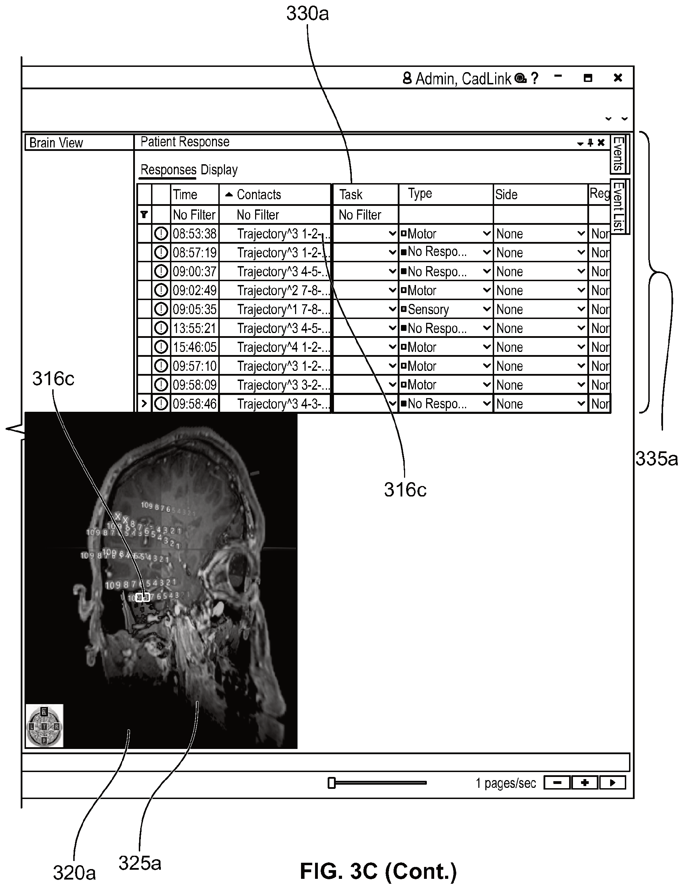

The system of the present specification also affords navigation from electrophysiological data to anatomical correlations. As shown in the unified viewing environment 300c of FIG. 3C, user interactions in the EEG display trigger corresponding highlights in the 3D model and contact lists. If the clinician selects (touch or click), hovers over, or moves the mouse cursor over an individual EEG trace 318c in the first view area 305a, the module 125 automatically highlights the associated one or more electrode contacts 316c within the 3D brain model 325a in the second view area 320a. Concurrently, these same associated one or more electrode contacts 316c are also automatically selected or highlighted in the list within the third view area 330a. Further, if the clinician selects (touch or click), hovers over, or moves the mouse cursor over an electrode block 312a, which represents a group of traces from a single electrode, in the first view area 305a, the entire associated electrode and all of its contacts are automatically highlighted in the 3D brain model 325a in the second view area 320a. The selection is also highlighted in the list in the third view area 330a.

In addition, if the clinician selects (touch or clicks), hovers over, or moves the mouse cursor over an electrode contact within the list in the third view area 330a, the associated electrode contact is automatically highlighted in the 3D brain model 325a in the second view area 320a and the associated EEG trace is also automatically highlighted in the first view area 305a.

If the clinician selects multiple EEG traces (e.g. by using Ctrl+Click) which may or may not be contiguous, in the first view area 305a, the associated electrode contacts are automatically highlighted in the 3D brain model 325a in the second view area 320a and in the list 335a of electrode contacts in the third view 330a. In this embodiment, the clinician can use a multi-trace selection tool to select multiple EEG traces from the first view area 305a, which can be adjacent and vertically stacked atop each other (contiguous) or may have a substantial degree of separation (non-contiguous). After selection, the clinician is automatically presented with which electrode contacts in both the 3D brain model 325a and the contact list 335a correspond to each selected EEG trace. This allows the clinician to visualize, for any number of selected EEG traces, how they map to specific brain regions via their associated electrode contacts.

At step 214, the clinician identifies an abnormal EEG event or event-of-interest (such as a spike event, abnormal EEG event, and seizure activity or seizure pattern) by analyzing at least one EEG trace displayed in the first view area. Upon selecting, hovering over, or clicking the at least one EEG trace (by the clinician), the module 125 automatically highlights the corresponding at least one electrode contact in the 3D brain model shown in the second view area. For events involving multiple traces, the clinician can select them simultaneously (for example, via Ctrl+Click), triggering collective highlighting of all associated contacts in the 3D model. This correlates temporal EEG data with spatial anatomical context, enabling rapid linkage between electrical anomalies and their potential neuroanatomical origins.

At step 216, the clinician selects the at least one electrode contact associated with the at least one EEG trace, then activates an ROI montaging function - for example, by clicking a graphical viewing element or icon within the unified viewing environment. The clinician then specifies or outlines an ROI around the chosen at least one electrode contact. In some implementations, the selected contact is visually highlighted using a predetermined color. If multiple electrode contacts are highlighted due to the selection of several EEG traces linked to an abnormal event, the clinician may further select a specific contact of interest from among the highlighted group before enabling the ROI montaging function. In certain cases, this contact of interest may be centrally located within the group of highlighted contacts.

In some embodiments, upon activation of the ROI montaging function, the module 125 provides the clinician with a ROI or volume capture tool. This tool allows the clinician to manually create, expand, or contract a 3D geometric shape (such as a sphere) around the contact of interest, automatically selecting any additional contacts that fall within this shape. The size and position of the geometric shape define the ROI and determine which contacts are auto-selected. The module 125 is configured to automatically highlight all contacts within the defined shape - regardless of their electrode origin - along with any associated EEG data from those contacts. Thus, highlighted contacts may span across multiple electrodes, not just a single one.

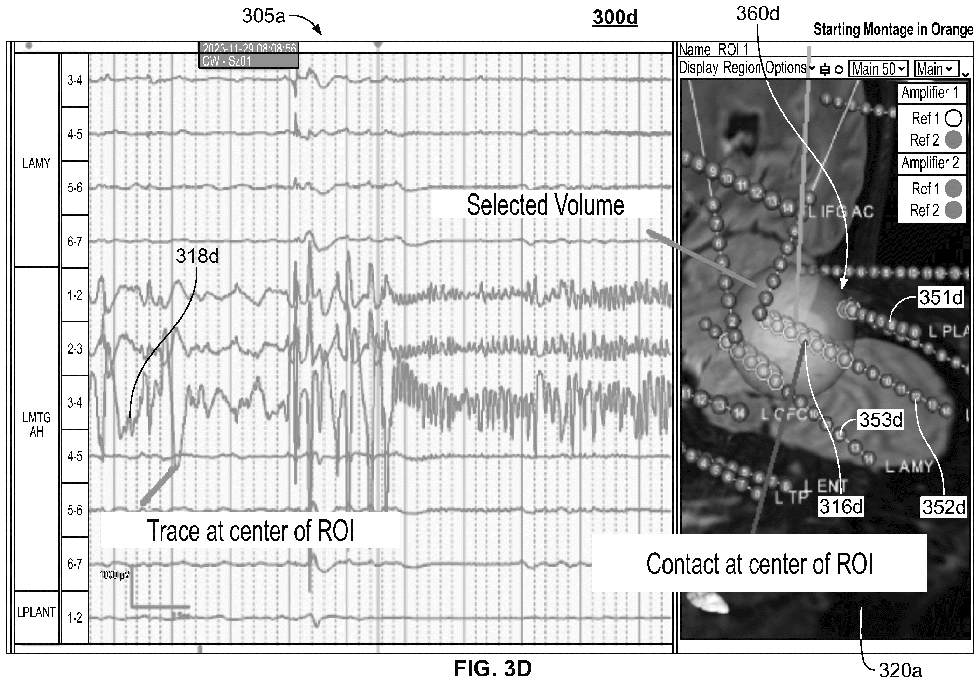

As illustrated in the unified viewing environment 300d of FIG. 3D, a plurality of bipolar montages has been generated using adjacent electrode contacts, within first electrode 351d, second electrode 352d, and third electrode 353d (for example, following execution of step 206). In other embodiments, the montages may be configured as referential. The clinician identifies an abnormal event by referencing at least one EEG trace 318d. To interact with the trace, the clinician may click, point to, hover over, or move the mouse cursor across the EEG trace 318d within the first view area 305a. This interaction triggers the automatic highlighting of one or more associated electrode contacts 316d in the second view area 320a.

The clinician then activates the Region of Interest (ROI) montaging function, prompting the module 125 to provide access to the ROI or volume capture tool. Using this tool, the clinician can manually define a 3D geometrical shape 360d, such as a sphere, around the highlighted electrode contact(s) 316d. This shape can be expanded or collapsed and serves to automatically include additional contacts located within its volume, regardless of their associated electrodes - that is, the automatically included contacts may be positioned within an electrode and/or across electrodes. The size and positioning of the shape define the ROI, thereby determining which additional contacts are auto-selected based on their spatial inclusion within the defined region.

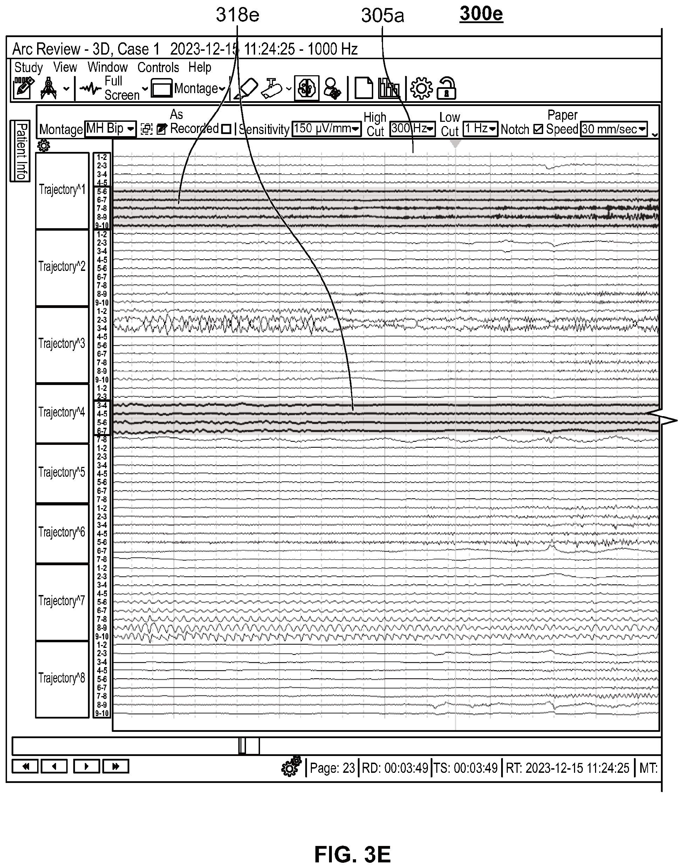

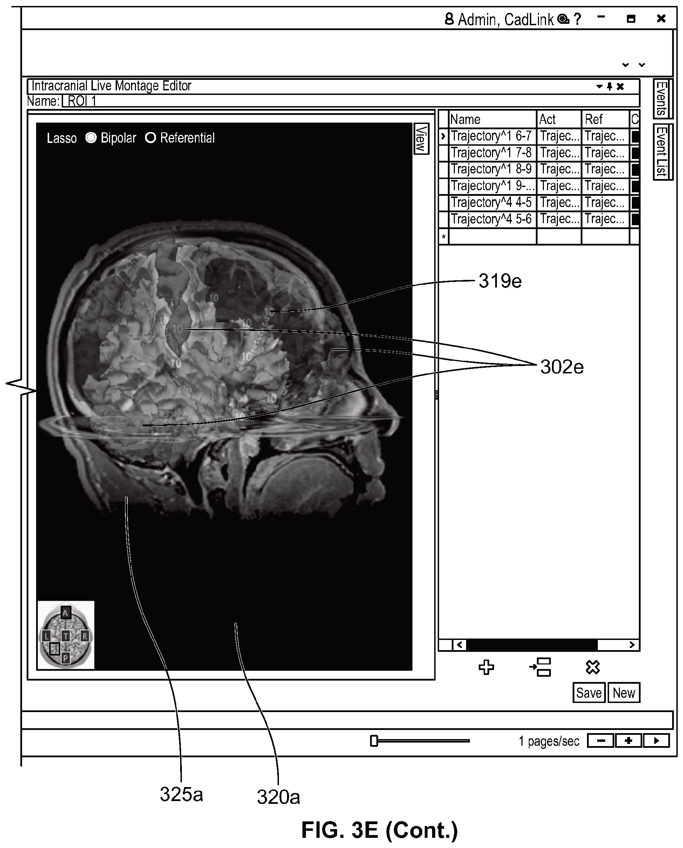

In some embodiments, the module 125 is configured to integrate a predefined anatomical atlas into the 3D brain model. FIG. 3E illustrates a unified viewing environment 300e that displays an anatomical atlas within the 3D brain model 325a, in accordance with some embodiments of the present specification. The anatomical atlas is used to illustrate anatomical regions 302e, in the second view area 320a, that may be differentiated from one another and overlaid upon the 3D brain model 325a. In one embodiment, the regions 302e may be differentiated based on color-coding, using a predefined color-scale. In response to a clinician selecting at least one of the anatomical regions 302e, all electrode contacts 319e within the at least one selected anatomical region across electrodes are automatically highlighted alongside their EEG traces 318e in the first view area 305a. In some embodiments, individual contacts can be included/excluded to refine and tailor the montage to further examine and define an anatomical and/or functional region.

In alternative embodiments, the ROI can be determined using an event detection algorithm, such as a spike detector, which is a tool (software-based or algorithmic) that automatically identifies sharp waves (spikes) in EEG recordings. These spikes represent transient electrical events/abnormalities or regions of interest based on detected neural activity patterns. EEG spike detectors use signal processing and machine learning to analyze data for characteristic features of the spike. A spike detection algorithm searches for electrical activity by dividing EEG data into small time windows, searching for local maxima within these windows and applying criteria such as duration and amplitude thresholds, evaluating the shape and spread of potential spikes, and optionally grouping the spikes.

Referring back to FIG. 2, at step 218, based on the clinician-specified ROI, the module 125 generates and displays ROI-specific montages using the electrode contacts that have been highlighted or selected within the specified ROI. In various embodiments, the module 125 may generate multiple montage types, including, for example, referential and bipolar configurations.

### Head Modeling

At step 220, the module 125 generates a patient-specific head model derived from the 3D brain model reconstruction, integrating virtual electrodes and contacts and accounting for volume conductivity-the electrical properties of brain tissues, skull, and cerebrospinal (CSF) fluid. This model enables precise simulation of electrical propagation across brain tissues by either assuming uniform tissue conductivity or using MRI data to assign tissue-specific conductivity values, thereby improving accuracy for dipole localization and montage optimization in EEG analysis.

### Lead field matrix calculation

At step 222, the module 125 uses the head model to compute a lead field matrix, a mathematical construct that represents the electric potential generated at each selected electrode contact within the ROI by a unit dipole located at a specific position and orientation in the source space. In other words, the lead field matrix serves as a mapping between the brain's source space (dipole sources) and the sensor space (electrodes and contacts), translating neural activity into measurable electrical signals. This computation forms part of the forward modeling process, expressed by the equation: V = L . J, where: V is the vector of voltages measured at the electrodes, L is the lead field matrix, and J is the vector of dipole source amplitudes.

### Dipole Modeling As An Inverse Solution

In accordance with aspects of the present specification, an equivalent, best-fitting or most likely vector dipole source responsible for the observed abnormal EEG activity is estimated using inverse solution techniques. These methods use the recorded electrode voltages (sensor space) in conjunction with the lead field matrix to infer the underlying distribution of dipole sources within the brain. Common inverse solution techniques, known to those of ordinary skill in the art, include Minimum Norm Estimate (MNE), Low-Resolution Brain Electromagnetic Tomography (LORETA), beamforming, least-squares minimization, and various Bayesian approaches such as Variational Bayesian methods and sparse prior models.

In one embodiment, the following steps outline a method for solving the inverse problem. At step 224, the module 125 analyzes EEG recordings during an abnormal event (for example, a spike or seizure activity) and identifies data corresponding to the highest-amplitude EEG voltages, which are indicative of proximity to the neural source. The signal-to-noise ratio is best for contacts closest to the event and allow for the best estimation of the generating dipole, since these contact(s) will usually have the largest amplitude spike voltage. As described above, a spike detector is used to define a region of interest, which is then displayed on all contacts with amplitudes that drop rapidly with distance. Using the highest-amplitude EEG voltages, the module 125 generates an initial estimate of a vector dipole source. The estimated dipole is superimposed on the patient 3D brain model within the 3D brain model unified viewing environment so that it can be visualized and evaluated in an anatomical context.

The vector dipole source is characterized by six parameters or dimensions that collectively define its spatial location or position, orientation, and amplitude within the patient's 3D brain model. The dipole's spatial location is determined by its x, y and z Cartesian coordinates, representing the origin of neural activity or the dipole's exact location. The orientation of the dipole is defined by two angular coordinates: a first angular coordinate indicative of the yaw (azimuth), which is rotation around the vertical (z) axis, indicating the left-right direction in the horizontal plane and a second angular coordinate indicative of the pitch (elevation), which is rotation around the side-to-side (y) axis, determining the upward or downward tilt of the dipole. The sixth parameter, amplitude, is representative of the strength of the dipole moment, indicating the magnitude of neural activity at the source. Together, the six parameters or dimensions describe a vector dipole source in 3D space in terms of: location, or where neural activity originates, direction of current flow (as neural activity has a directional component), and amplitude (the intensity of the neural current). In some embodiments, the dipole model excludes an angular coordinate for roll (rotation around the front-to-back or x-axis), as it does not affect the resulting field for the purposes of computation.

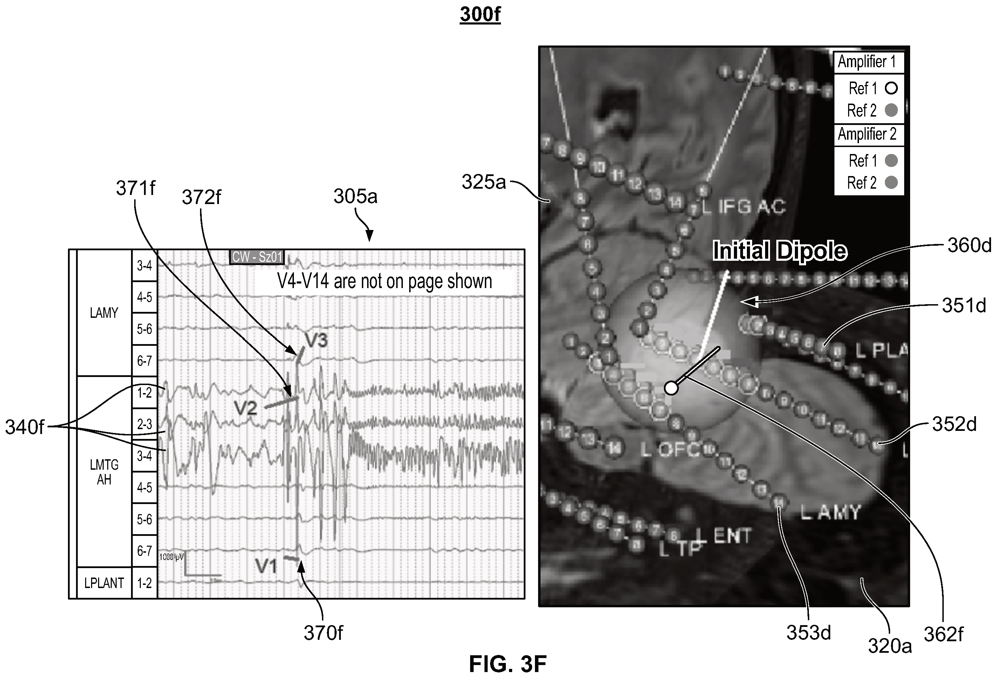

As illustrated in the unified viewing environment 300f of FIG. 3F, the module 125 is configured to generate an initial vector dipole source 362f using a set of the highest-amplitude EEG voltages, specifically, voltages 370f, 371f, and 372f. It should be noted that voltage 370f is of opposite polarity from voltages 371f and 372f, and therefore the dipole is located at a position between the two contacts. These highest-amplitude EEG voltages correspond to a subset of EEG traces 340f, displayed in the first view area 305a, which are associated with an abnormal event, suspected spike, or event of interest. In some embodiments, the EEG traces 340f are derived from ROI montages composed of multiple electrode contacts located within the first electrode 351d, second electrode 352d, and third electrode 353d. These contacts are highlighted within the 3D brain model 325a in the second view area 320a and fall within the defined 3D geometrical shape 360d representing the Region of Interest (ROI).

At step 226, the module 125 employs the lead field matrix to calculate the theoretical voltages that the vector dipole source would produce at each of the selected electrode contacts within the ROI.

At step 228, the module 125 calculates a composite error value by comparing the theoretical voltages, derived from the vector dipole source via the lead field matrix as in step 226, with the actual recorded EEG voltages associated with the suspected spike or event of interest at each selected electrode contact within the ROI.

At step 230, the module 125 initiates an iterative process in which it makes small adjustments to the six parameters (that is, the dimensions) defining the vector dipole source, namely its 3D position, orientation (yaw and pitch), and amplitude. For each new set of parameter values, a corresponding composite error value is recalculated. At step 232, the iterative adjustment of step 230 continues in a direction that reduces the composite error value. The process is repeated for each of the six parameters until the composite error is minimized (that is, until a minimum composite error is determined), identifying a vector dipole configuration that best fits the recorded EEG data. In embodiments, the number of iterations varies with the accuracy of the initial estimate, and typically ranges from 50-100. In embodiments, the composite error is computed by computing the voltage at each contact based on the current dipole estimate, subtracting the computed voltage from the measured voltage to generate an error at each contact, and combining the errors across the contacts using a linear summation or root mean square (RMS) calculation. In embodiments, the error reaches a minimum, and in still other embodiments, there may be more than one minimum.

Thus, steps 224 through 232 implement an optimization technique aimed at refining the six defining parameters of the vector dipole source, its location, direction, and strength, so that the predicted voltages from the forward model align as closely as possible with the actual measured EEG voltages from the suspected spike or event of interest. In essence, the optimized parameters of the estimated vector dipole source represent the most likely neural origin of the observed EEG activity. Stated differently, the six parameters or dimensions of the estimated best-fitting vector dipole source define the likely neural source of the actual measured/recorded EEG voltages corresponding to the suspect spike event or event-of-interest. At step 234, the optimized, best-fitting vector dipole source is displayed within the 3D brain model in the unified viewing environment.

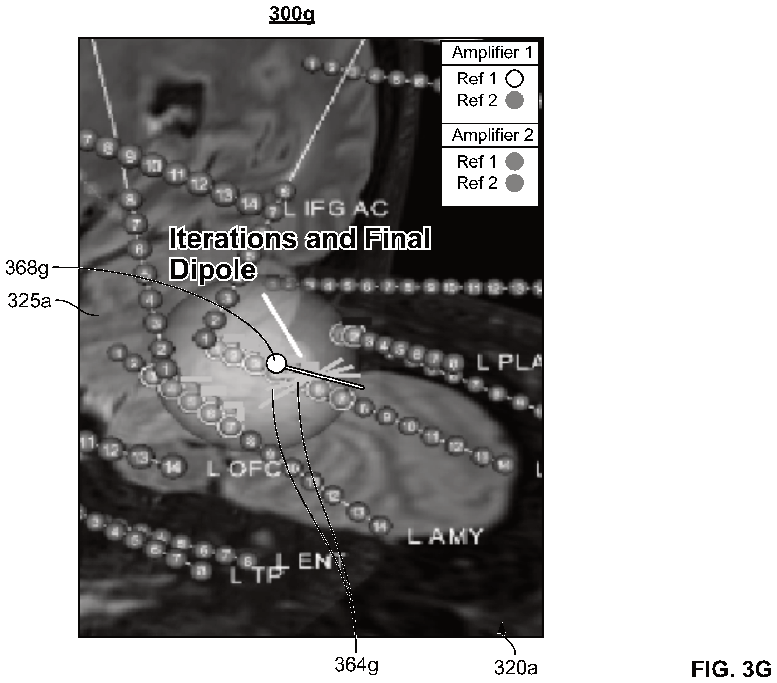

Referring to FIG. 3G, the unified viewing environment 300g displays a series of interim vector dipole sources 364g, which were generated during the iterative optimization process described in step 230. Also shown is the final estimated dipole source 368g, representing the equivalent, best-fitting, or most likely source that corresponds to the actual recorded EEG voltages associated with the abnormal event (for example spike or seizure activity). Both the interim dipoles 364g and the final dipole 368g are displayed within the 3D brain model 325a in the second view area 320a.

At step 236, the module 125 evaluates the accuracy of the estimated dipole by calculating a goodness-of-fit (GOF) metric, which quantifies how closely the dipole's simulated voltages align with the recorded EEG data during the abnormal event. In other words, the GOF is the remaining error after the solution converges to a minimum error value.

### First Re-Montaging

The computed equivalent, best-fitting, or most likely vector dipole source representing the abnormal event (for example spike or seizure activity), is positioned and oriented within the 3D brain model. However, because the recording contacts are distributed in an irregular spatial pattern (any two electrodes are not, in general, in a single plane or evenly spaced), interpreting the resulting EEG traces, which are from the contact montages nearest the dipole, can be challenging. To aid in this interpretation, the module 125 generates both first and second sets of montages.

At step 238, the module 125 automatically generates a first set of montages based on a subset of electrode contacts located in the vicinity of the computed equivalent, best-fitting, or most likely vector dipole source within the 3D brain model. In embodiments, the system (optionally via module 125) is configured to select the contact with the largest voltage as the center of a spherical volume that encompasses on the order of 6 total contacts, preferably with at least one contact in each of six half-planes around the center. The first set of montages is displayed within the 3D brain model, and the corresponding first plurality of EEG traces are presented either in the first view area or, alternatively, in a third, distinct view area within the integrated viewing environment. In some embodiments, the subset of electrode contacts is drawn from the initially selected electrode contacts within the ROI and is further refined to include only those in close proximity to the best-fitting vector dipole source.

In certain embodiments, the first set of montages reconfigures the EEG signals (which, in some embodiments, are originally bipolar and, therefore, referenced to a single reference) into bipolar or differential montages composed of electrode contact pairs located near the best-fit (computed) vector dipole source. The first set of montages may include pairs formed across different electrodes (cross-electrode montages), providing a more localized and interpretable view of the EEG activity surrounding the identified source.

### Virtual Electrode Contacts Modeling and Creation of Second Set of Montages

In some embodiments, virtual contacts, or virtual sensors, are generated subsequent to dipole modeling or source localization. These virtual contacts represent neural activity projected onto locations within the brain where no physical electrodes or contacts are implanted. In other words, virtual contact modeling enables the reconstruction of neural signals originating from the estimated equivalent, best-fitting, or most likely vector dipole source at brain locations that lack direct physical recordings. This reconstruction is based on the signals captured by nearby physical electrode contacts.

Thus, at step 240, the module 125 uses the computed (best-fit) vector dipole source to generate a set of virtual contacts, which are displayed within the 3D brain model. In some embodiments, these virtual contacts are arranged in a regularly spaced grid along two axes of a 2D virtual plane, centered on the x, y, z coordinates of the estimated vector dipole source location. This arrangement allows for consistent spatial sampling of the neural activity surrounding the modeled source.

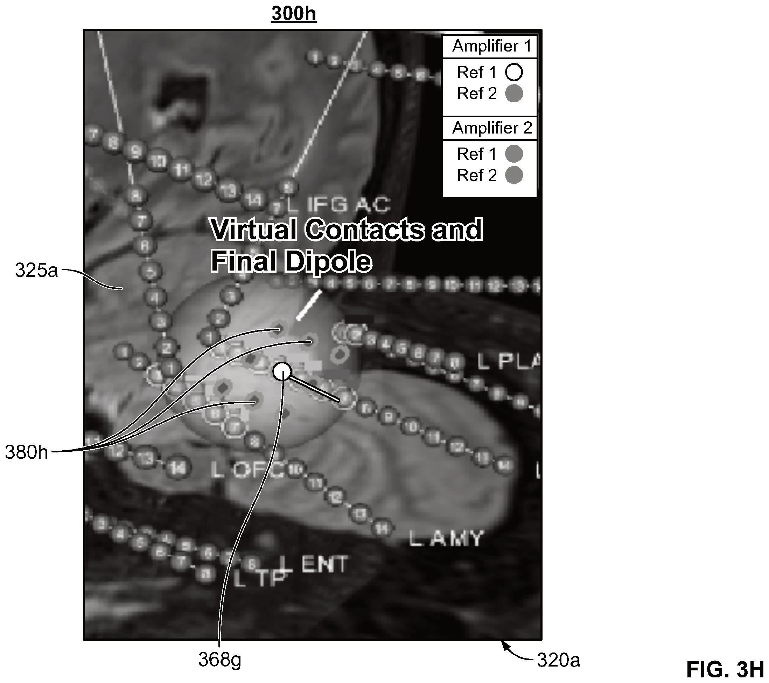

Referring to FIG. 3H, the integrated viewing environment 300h displays a plurality of virtual contacts 380h within the 3D brain model 325a, shown in the second viewing area 320a. Also visualized is the estimated equivalent, best-fitting, or most likely vector dipole source 368g, which best matches the actual recorded EEG voltages associated with the abnormal event, suspected spike, or event of interest.

### Computing Voltages at the Plurality of Virtual Contacts

At step 242, the module 125 determines the voltages at each of the virtual contacts by extrapolating from the voltages recorded at nearby physical electrode contacts-specifically, those located in the vicinity of the computed equivalent, best-fitting, or most likely vector dipole source.

In one embodiment, the voltage at a virtual contact is computed based on the dipole model. The voltage is proportional to the dipole's amplitude, modified by the geometric relationship between the dipole and the virtual contact location. Specifically, the voltage at a virtual contact is given by the formula: virtual contact voltage = (dipole voltage / r³) × (1 + cos(α)), where r is the distance between the dipole and the virtual contact, α is the angle between the dipole orientation and the line connecting the dipole to the virtual contact, and all calculations are performed in the same 3D coordinate space. In embodiments, the voltage at each point (virtual contact) is considered a referential signal, which can then be used to create a bipolar signal between contacts as well.

Presented below is an exemplary set of programmatic code or instructions which, when executed, cause the module 125 to determine the voltages at each of the virtual contacts:

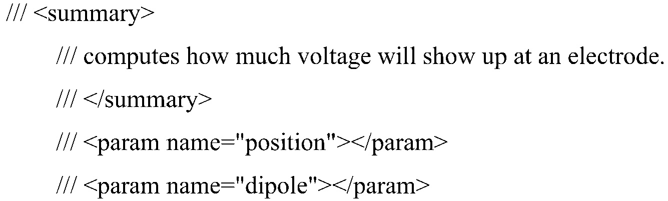

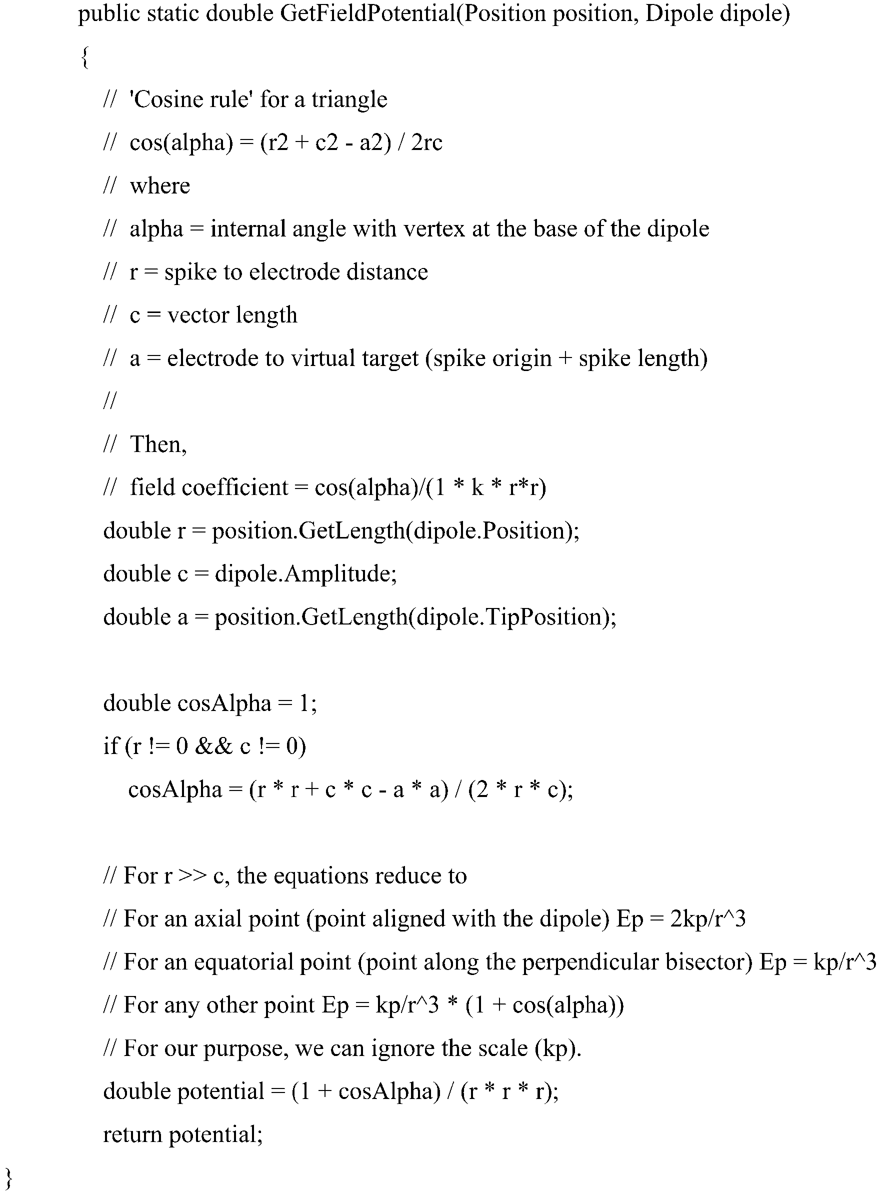

At step 244, the module 125 automatically generates and displays a second set of montages based on the plurality of virtual contacts. A corresponding second plurality of EEG traces are also displayed either in the first view area or, alternatively, in a third view area distinct from the first, within the integrated viewing environment. This results in a planar, grid-like montage set. Notably, the 2D virtual plane representation of the virtual electrodes-and thus the second set of montages-closely resembles the conventional 2D montages used in the 10-20 system of extracranial EEG, providing intuitive interpretability. In embodiments, the voltage at each point (virtual contact) is considered a referential signal, which can then be used to create a bipolar signal between contacts as well.

In some embodiments, the virtual electrodes form a 3 × 3 array, enabling montages that display signals oriented along both the x-axis and y-axis.

When multiple spike events originate from the same brain locus, the corresponding EEG signals may be averaged either before or after analysis. Alternatively, multiple vector dipoles may be visualized within the 3D brain model to illustrate event variability and the influence of noise on the calculations. Because spike events may evolve over time, the position and orientation of the vector dipole are captured sequentially, allowing visualization of the spike's propagation within the 3D brain model.

The above examples are merely illustrative of the many applications of the systems and methods of the present specification. Although only a few embodiments of the present invention have been described herein, it should be understood that the present invention might be embodied in many other specific forms without departing from the spirit or scope of the invention. Therefore, the present examples and embodiments are to be considered as illustrative and not restrictive, and the invention may be modified within the scope of the appended claims.

## Claims

1. A computer-implemented method of enabling a user to generate one or more montages using a computing device, wherein the computing device comprises at least one processor configured to execute programmatic instructions and a non-transient memory adapted to store the programmatic instructions, comprising:
generating a graphical user interface, wherein the graphical user interface comprises at least a first display area and a second display area, wherein the first and second display areas are separate and positioned side-by-side;
acquiring, using the computing device, post-surgical data, wherein the post-surgical data comprises a three-dimensional image of a patient's brain and comprises data indicative of a visual representation of one or more electrodes placed in the patient's brain, wherein each of the one or more electrodes has associated one or more contacts therewith;
acquiring, using the computing device, EEG data of the patient, wherein the EEG data comprises a first plurality of EEG traces;
simultaneously displaying the EEG data in the first display area and the post-surgical data in the second display area of the graphical user interface;
automatically visually associating the visual representation of each of the one or more electrodes and associated one or more contacts in the post-surgical data with a corresponding one of the first plurality of EEG traces;
receiving a user selection of at least one EEG trace of the first plurality of EEG traces;
in response to said receiving of the user selection of the at least one EEG trace, causing at least one contact of the one or more contacts to be automatically selected in the second display area, wherein said automatically selected at least one contact is associated with said user-selected at least one EEG trace;
receiving a user selection of a region of interest around the automatically selected at least one contact;
in response to said receiving of the user selection of the region of interest, causing a first plurality of contacts, within the region of interest, to be automatically selected;
automatically generating a first set of montages based on the automatically selected first plurality of contacts; and
displaying the automatically generated first set of montages in the first display area.

2. The computer-implemented method of claim 1, wherein the three-dimensional image is co-registered with a three-dimensional coordinate system

3. The computer-implemented method of claim 1 or 2, further comprising automatically computing a lead field matrix using a head model.

4. The computer-implemented method of claim 3, further comprising generating the head model by associating a volume conductivity of the patient's brain with the three-dimensional image of the patient's brain, or wherein the lead field matrix defines an electric potential that a unit dipole would produce at each of the first plurality of contacts.

5. The computer-implemented method of claim 3 or 4, further comprising automatically generating an initial vector dipole source using data indicative of a first plurality of voltages associated with said at least one EEG trace of the first plurality of EEG traces.

6. The computer-implemented method of claim 5, further comprising automatically determining a plurality of expected voltages generated by the initial vector dipole source at at least a portion of the first plurality of contacts within the region of interest.

7. The computer-implemented method of claim 6, further comprising automatically computing a computed dipole source based on said plurality of expected voltages.

8. The computer-implemented method of claim 7, further comprising determining the computed vector dipole source by:
generating a composite error value by comparing the plurality of expected voltages with a plurality of actual voltages at the at least a portion of the first plurality of contacts within the region of interest;
iteratively changing dimensions of the initial vector dipole source to generate successive vector dipole sources; and
iteratively generating a successive composite error value for each of the successive vector dipole sources in a direction of decreasing composite error value until a minimum composite error is determined, wherein the computed vector dipole source corresponds to the minimum composite error, optionally wherein each of the initial vector dipole source and the computed vector dipole source is associated with a position, an orientation and an amplitude.

9. The computer-implemented method of claim 7, wherein at least one of:
the computed vector dipole source is displayed in the second display area; or
further comprising automatically generating a plurality of virtual contacts based on the computed dipole source, wherein the plurality of virtual contacts are displayed in the second display area.

10. The computer-implemented method of claim 9, further comprising automatically generating a second set of montages based on the plurality of virtual contacts, wherein the second set of montages is displayed in the second display area, and wherein a second plurality of EEG traces, corresponding to the second set of montages, is displayed in the first display area.

11. The computer-implemented method of any preceding claim, wherein the selected at least one EEG trace corresponds to an abnormal event, or further comprising enabling the region of interest to be specified by presenting a tool adapted to draw a 3D geometrical shape around the automatically selected at least one contact, and wherein at least a size of the 3D geometrical shape determines automatic selection of the first plurality of contacts, optionally wherein the first plurality of contacts is positioned within an electrode and/or across electrodes.

12. The computer-implemented method of claim 8, further comprising:
automatically generating a third set of montages based on a third plurality of contacts positioned in the vicinity of the computed dipole source; and
displaying a third plurality of EEG traces corresponding to the third set of montages in the first display area.

13. A computer-implemented method of enabling a user to generate one or more montages using a computing device, wherein the computing device comprises at least one processor configured to execute programmatic instructions and a non-transient memory adapted to store the programmatic instructions, comprising:
generating a graphical user interface, wherein the graphical user interface comprises at least a first display area and a second display area, wherein the first display area is separate and distinct from the second display area, and wherein the first and second display areas are positioned side-by-side;
acquiring, using the computing device, post-surgical data, wherein the post-surgical data comprises a three-dimensional image of a patient's brain and comprises data indicative of a visual representation of one or more electrodes surgically placed in the patient's brain, wherein each of the one or more electrodes has associated one or more contacts, and wherein the three-dimensional image is co-registered with a three-dimensional coordinate system;
acquiring, using the computing device, EEG data of the patient;
displaying, simultaneously, the EEG data in the first display area and the post-surgical data in the second display area of the graphical user interface, wherein the EEG data is displayed as a first plurality of EEG traces;
automatically visually associating the visual representation of each of the one or more electrodes and associated one or more contacts in the post-surgical data with a corresponding one of the first plurality of EEG traces;
automatically selecting at least one contact in the second display area in response to receiving user data indicative of at least one EEG trace of the first plurality of EEG traces, wherein said automatically selected at least contact is associated with said selected at least one EEG trace;
automatically selecting a first plurality of contacts, within the region of interest, in response to receiving user data indicative of a region of interest around the automatically selected at least one contact;
automatically generating a first set of montages based on the first plurality of contacts;
automatically computing a lead field matrix using the head model;
automatically generating an initial vector dipole source using data indicative of a first plurality of voltages associated with said at least one EEG trace of the first plurality of EEG traces;
automatically determining a plurality of expected voltages generated by the initial vector dipole source at at least a portion of the first plurality of contacts within the region of interest;
automatically computing a computed dipole source based on said plurality of expected voltages;
automatically generating a plurality of virtual contacts based on the computed vector dipole source, wherein the plurality of virtual contacts are displayed in the second display area; and
automatically generating a second set of montages based on the plurality of virtual contacts and displaying a second plurality of EEG traces, corresponding to the second set of montages, in the first display area.

14. The computer-implemented method of claim 13, further comprising automatically generating a third set of montages based on a third plurality of contacts positioned in a vicinity of the computed vector dipole source, or wherein the lead field matrix defines an electric potential that a unit dipole would produce at each of the first plurality of contacts.

15. The computer-implemented method of claim 13, wherein each of the initial vector dipole source and the computed vector dipole source is associated with a position, an orientation and an amplitude, or further comprising generating the head model by associating a volume conductivity of the patient's brain with the three-dimensional image of the patient's brain.
